# EUROPEAN PATENT SPECIFICATION

(11) **EP 3 936 038 B1**
(45) Date of publication and mention of the grant of the patent: **18.03.2026**
(21) Application number: 20193972.5
(22) Date of filing: 01.09.2020
(51) Int. Cl.: A61B 5/024, A61B 5/0245

(54) **SIGNAL PROCESSING METHOD, APPARATUS AND NON-TRANSITORY COMPUTER READABLE STORAGE MEDIUM FOR CALCULATING HEART RATES**
SIGNALVERARBEITUNGSVERFAHREN, VORRICHTUNG UND NICHT-TRANSITORISCHES COMPUTERLESBARES SPEICHERMEDIUM ZUR BERECHNUNG DER HERZFREQUENZ
PROCÉDÉ DE TRAITEMENT DE SIGNAL, APPAREIL ET SUPPORT D'ENREGISTREMENT LISIBLE PAR ORDINATEUR NON TRANSITOIRE POUR LE CALCUL DES FRÉQUENCES CARDIAQUES

(30) Priority: 10.07.2020 TW 109123292
(43) Date of publication of application: 12.01.2022
(73) Proprietor: Wistron Corporation, New Taipei City 22181 (TW)
(72) Inventor: LIU, Zheng-De, 22181 New Taipei City (TW); HUANG, Kuo-Ting, 22181 New Taipei City (TW)
(74) Representative: 2K Patentanwälte Blasberg Kewitz & Reichel

(56) References cited:
- JP-A- 2009 261 419
- JP-A- H0 622 914
- US-A1- 2018 271 449
- US-A1- 2020 155 018

## Description

### BACKGROUND OF THE DISCLOSURE

### 1. Field of the Disclosure

The disclosure relates to a signal processing method, an apparatus and a non-transitory computer readable storage medium for calculating heart rates.

### 2. Description of Related Art

In recent years, with people's emphasis on health, various monitoring devices capable of monitoring heartbeats have appeared on the market. When the wearer is stable, the monitoring device can obtain the best physiological signal. When the wearer has a sudden movement (for example, turns the head, nods, gets up or the like), the monitoring device will produce noise to affect the accuracy. In the past, an acceleration signal of an accelerometer was used to determine whether the wearer had a movement. When the wearer is static, a peak value can be calculated smoothly, then an instant heart rate is calculated from a time difference between the adjacent peak values, and finally, an average heart rate is obtained by making statistics on several heart rates.
US2018271449A1 discloses a heartbeat detection system includes a Doppler sensor configured to receive a reflective wave from a subject, to obtain a Doppler signal; and a processor including a memory and a CPU. The processor executes applying a wavelet transform to the Doppler signal based on a plurality of scale factors, to obtain a wavelet coefficient for each of the scale factors; detecting a plurality of peaks of the wavelet coefficient for each of the scale factors; calculating each peak interval between the peaks next to each other, and each difference between the peak intervals next to each other, for each of the scale factors; selecting one of the scale factors that has a minimum variation in the differences of the peak intervals, as an optimal scale factor; and measuring the peak intervals calculated based on the optimal scale factor as heartbeat intervals.
US 2020155018A1 provides an information processing apparatus, information processing method, and program that make it possible to bring the measurement of the heart rate variability or pulse rate variability of a user freely moving around into a preferable state. There is an information processing apparatus including: a reliability degree calculation section that calculates a reliability degree of pulsation variability data or a body index; and a control unit that controls various kinds of processing on the basis of the calculated reliability degree. The pulsation variability data is acquired from sensing data acquired by a pulse wave sensor worn by a user. The body index is calculated from the pulsation variability data and indicates a physical state of the user. JP2009261419A provides a biological state estimating device highly accurately estimating a biological state even in a situation that it is difficult to highly accurately estimate the biological state while driving or the like, for instance.
In a step 200, whether an electrocardiographic sensor 1 and the hand of a driver are in contact with each other is decided. In a step 210, whether or not a heartbeat interval calculated from an electrocardiographic waveform is within a prescribed range is decided. When decided that the contact state with the electrocardiographic sensor 1 is not appropriate in the step 200 or when decided that the data of the heartbeat interval are abnormal in the step 210, whether or not a pulse wave sensor 3 and the hand of the driver are in contact with each other is decided in a step 230. In a step 240, whether or not a pulse wave interval calculated from a pulse wave waveform is within a prescribed range is decided. In a step 250, since a pulse interval is normal, the heartbeat interval is interpolated using the pulse rate interval, and the heartbeat interval is output to a storage device 17 and a display device 19.
JP 406022914A provides a heart rate detecting device which can surely detect the heart rate of a driver with high precision of a driver with high precision. Infrared ray type heart beat sensors 11 and 12 each of which has a light projection part which is installed on a steering wheel and projects infrared rays and a light receiving part for receiving the infrared ray and detects the heart rate of a driver from the pulsation of blood are provided. Further, an electric potential type heart beat sensor 16 is provided which is installed on the steering wheel 33 and is equipped with at least two electrodes and detects the heart rate of the driver from the electric potential difference due to the pulsation between both hands, and also a heart beat data processing means 18 is provided which compares the time sequence data quantity NDI of the heart rate detected by the infrared type heart beat sensors 11 and 12 and the time sequence data NDE of the heart rate detected by an electric potential type heart beat sensor 16, and adopts a larger value as an effective heart rate.

When the wearer has a sudden movement (for example, turns the head, nods, gets up or the like), signals sensed by the monitoring device will have noise, so it difficult to calculate the peak value. The current practice is to directly discard signals with noise. However, the instant heart rate is obtained from the adjacent peak values. If a signal is discarded, there will be a lack of two instant heart rates, which will cause a delay in the average heart rate.

### SUMMARY OF THE INVENTION

According to the invention there are provided a signal processing method, a signal processing apparatus and a non-transitory computer readable storage medium according to claims 1, 9 and 13, respectively, with advantageous embodiments being defined in the dependent claims 2-8 and 10-12.

### SUMMARY OF THE DISCLOSURE

The disclosure provides a signal processing method, an apparatus and a non-transitory computer readable storage medium, which can find out peak values from a physiological signal of undesirable quality, thereby stably outputting an average heart rate.

The signal processing method of the disclosure includes: taking out a plurality of segment signals based on a time series from a physiological signal; determining each of the segment signals as a desirable signal or an undesirable signal to calculate peak values of these segment signals determined as the desirable signals; in response to the i^{th} segment signal is determined as the undesirable signal, determining whether the (i-2)^{th} segment signal and the (i+2)^{th} segment signal are the desirable signals; under the condition of determining that both of the (i-2)^{th} segment signal and the (i+2)^{th} segment signal are the desirable signals, reserving the i^{th} segment signal determined as the undesirable signal to calculate a representative peak value of the i^{th} segment signal; and calculating a plurality of instant heart rates based on the segment signals determined as the desirable signals and the reserved segment signals determined as the undesirable signals.

In an embodiment of the disclosure, the step of under the condition of determining that both of the (i-2)^{th} segment signal and the (i+2)^{th} segment signal are the desirable signals, reserving the i^{th} segment signal to calculate the representative peak value of the i^{th} segment signal includes: determining a change trend of the i^{th} segment signal based on a first peak value of the (i-2)^{th} segment signal and a second peak value of the (i+2)^{th} segment signal; and obtaining the representative peak value of the i^{th} segment signal by using a calculation manner corresponding to the change trend.

In an embodiment of the disclosure, before the step of determining the change trend of the i^{th} segment signal, the method further includes: calculating an absolute value of a difference between the first peak value and the second peak value. Steps of determining the change trend of the i^{th} segment signal include: comparing the absolute value of the difference with a threshold value; if the absolute value of the difference is less than or equal to the threshold value, determining that the change trend of the i^{th} segment signal is a stable state; in response tothe absolute value of the difference is greater than the threshold value and the first peak value is less than the second peak value, determining that the change trend of the i^{th} segment signal is a continuous increase state; and in response tothe absolute value of the difference is greater than the threshold value and the first peak value is greater than the second peak value, determining that the change trend of the i^{th} segment signal is a continuous decrease state.

In an embodiment of the disclosure, the step of obtaining the representative peak value of the i^{th} segment signal by using the calculation manner corresponding to the change trend includes: when the change trend is the stable state, obtaining a line segment based on the first peak value and the second peak value; finding out a plurality of candidate peak values from the i^{th} segment signal; and taking out one of the candidate peak values closest to the line segment from these candidate peak values as the representative peak value of the i^{th} segment signal.

In an embodiment of the disclosure, the step of obtaining the representative peak value of the i^{th} segment signal by using the calculation manner corresponding to the change trend includes: under the condition of the change trend is the continuous increase state, obtaining a first line segment based on the first peak value and a third peak value of the (i-1)^{th} segment signal; obtaining a second line segment based on the second peak value and a fourth peak value of the (i+1)^{th} segment signal; finding out a plurality of candidate peak values from the i^{th} segment signal; taking out a first candidate peak value closest to the first line segment from these candidate peak values; taking out a second candidate peak value closest to the second line segment from these candidate peak values; in response to the first candidate peak value and the second candidate peak value are the same, taking the first candidate peak value as the representative peak value of the i^{th} segment signal; and in response tothe first candidate peak value and the second candidate peak value are different, taking the smallest of the first candidate peak value and the second candidate peak value as the representative peak value of the i^{th} segment signal.

In an embodiment of the disclosure, the step of obtaining the representative peak value of the i^{th} segment signal by using the calculation manner corresponding to the change trend includes: under the condition of the change trend is the continuous decrease state, obtaining a first line segment based on the first peak value and a third peak value of the (i-1)^{th} segment signal; obtaining a second line segment based on the second peak value and a fourth peak value of the (i+1)^{th} segment signal; finding out a plurality of candidate peak values from the i^{th} segment signal; taking out a first candidate peak value closest to the first line segment from these candidate peak values; taking out a second candidate peak value closest to the second line segment from these candidate peak values; in response tothe first candidate peak value and the second candidate peak value are the same, taking the first candidate peak value as the representative peak value of the i^{th} segment signal; and in response tothe first candidate peak value and the second candidate peak value are different, taking the largest of the first candidate peak value and the second candidate peak value as the representative peak value of the i^{th} segment signal.

In an embodiment of the disclosure, after determining whether the (i-2)^{th} segment signal and the (i+2)^{th} segment signal are the desirable signals in response tothe i^{th} segment signal is determined as the undesirable signal, the method further includes: under the condition of determining that at least one of the (i-2)^{th} segment signal and the (i+2)^{th} segment signal is the undesirable signal, discarding the i^{th} segment signal determined as the undesirable signal.

In an embodiment of the disclosure, the step of determining each of the segment signals as the desirable signal or the undesirable signal includes: determining the segment signals as the desirable signals or the undesirable signals by using a dynamic time warping (DTW) algorithm.

In an embodiment of the disclosure, the signal processing method further includes: after obtaining the representative peak value of the i^{th} segment signal, obtaining a comparison value based on an average value of a first peak value of the (i-2)^{th} segment signal and a second peak value of the (i+2)^{th} segment signal; determining whether the representative peak value is greater than the comparison value; in response tothe representative peak value is greater than the comparison value, determining that the i^{th} segment signal is unreliable, and discarding the i^{th} segment signal; and in response tothe representative peak value is less than or equal to the comparison value, determining that the i^{th} segment signal is reliable, and reserving the i^{th} segment signal.

The signal processing apparatus of the disclosure includes: a heart rate sensor, sensing a physiological signal of a wearer; a storage unit, including a plurality of code segments; and a processor, coupled to the heart rate sensor and the storage unit. The processor executes the code segments to: receive the physiological signal from the heart rate sensor; take out a plurality of segment signals based on a time series from the physiological signal; determine each of the segment signals as a desirable signal or an undesirable signal to calculate peak values of these segment signals determined as the desirable signals; in response tothe i^{th} segment signal is determined as the undesirable signal, determine whether the (i-2)^{th} segment signal and the (i+2)^{th} segment signal are the desirable signals; under the condition of determining that both of the (i-2)^{th} segment signal and the (i+2)^{th} segment signal are the desirable signals, reserve the i^{th} segment signal to calculate a representative peak value of the i^{th} segment signal; when determining that at least one of the (i-2)^{th} segment signal and the (i+2)^{th} segment signal is the undesirable signal, discard the i^{th} segment signal; and calculate a plurality of instant heart rates based on the segment signals determined as the desirable signals and the reserved segment signals determined as the undesirable signals.

The non-transitory computer readable storage medium of the disclosure records at least one programmed instruction. After being loaded into an electronic device, the at least one programmed instruction performs the following steps: taking out a plurality of segment signals based on a time series from a physiological signal; determining each of the segment signals as a desirable signal or an undesirable signal to calculate peak values of these segment signals determined as the desirable signals; in response tothe i^{th} segment signal is determined as the undesirable signal, determining whether the (i-2)^{th} segment signal and the (i+2)^{th} segment signal are the desirable signals; when determining that both of the (i-2)^{th} segment signal and the (i+2)^{th} segment signal are the desirable signals, reserving the i^{th} segment signal to calculate a representative peak value of the i^{th} segment signal; under the condition of determining that at least one of the (i-2)^{th} segment signal and the (i+2)^{th} segment signal is the undesirable signal, discarding the i^{th} segment signal; and calculating a plurality of instant heart rates based on the segment signals determined as the desirable signals and the reserved segment signals determined as the undesirable signals.

Based on the above, the disclosure can further determine whether to reserve the undesirable signals to find out the peak values from the undesirable signals, thereby stably outputting the average heart rate.

### BRIEF DESCRIPTION OF THE DRAWINGS

FIG. 1 is a block diagram of a signal processing apparatus according to an embodiment of the disclosure.
FIG. 2 is a flow chart of a signal processing method according to an embodiment of the disclosure.
FIG. 3A is a schematic diagram of a physiological signal according to an embodiment of the disclosure.
FIG. 3B is a schematic diagram of calculating instant heart rates according to an embodiment of the disclosure.
FIG. 4 is a waveform diagram of a stable state according to an embodiment of the disclosure.
FIG. 5 is a waveform diagram of a continuous increase state according to an embodiment of the disclosure.
FIG. 6 is a waveform diagram of a continuous decrease state according to an embodiment of the disclosure.
FIG. 7 is a waveform diagram of a calculation manner of a representative peak value in a stable state according to an embodiment of the disclosure.
FIG. 8A to FIG. 8B are waveform diagrams of a calculation manner of a representative peak value in a continuous increase state according to an embodiment of the disclosure.
FIG. 9A to FIG. 9B are waveform diagrams of a calculation manner of a representative peak value in a continuous decrease state according to an embodiment of the disclosure.

### DESCRIPTION OF THE EMBODIMENTS

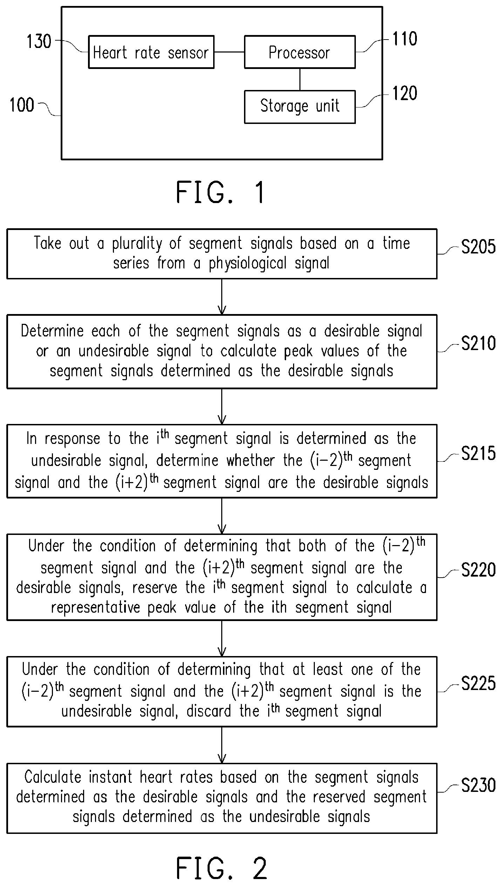

FIG. 1 is a block diagram of a signal processing apparatus according to an embodiment of the disclosure. Referring to FIG. 1, the signal processing apparatus 100 includes a processor 110, a storage unit 120 and a heart rate sensor 130. The processor 110 is directly or indirectly electrically coupled to the storage unit 120 and the heart rate sensor 130.

The processor 110 is, for example, a central processing unit (CPU), a physical processing unit (PPU), a programmable microprocessor (microprocessor), an embedded control chip, a digital signal processor (DSP), an application specific integrated circuit (ASIC) or other similar devices.

The storage unit 120 is, for example, any type of fixed or movable random access memory (RAM), read-only memory (ROM), flash memory, hard disk, or other similar devices or a combination of these devices. A plurality of code segments are stored in the storage unit 120. After the above code segments are installed, the processor 110 executes the code segments to realize the following signal processing method.

The heart rate sensor 130 is configured to sense a physiological signal of a wearer. The heart rate sensor 130 is, for example, a sensor using photoplethysmography (PPG), but is not limited thereto, and may be, for example, a radar sensor or an electrocardiogram (ECG) sensor.

FIG. 2 is a flow chart of a signal processing method according to an embodiment of the disclosure. In the embodiment, the processor 110 is configured to execute the code segments stored in the storage unit 120, thereby realizing the following signal processing method.

In step S205, a plurality of segment signals are taken out based on a time series from a physiological signal. The processor 110 collects the physiological signal within a sampling time range through the heart rate sensor 130, and divides the physiological signal into the plurality of segment signals. Next, in step S210, each of the segment signals is determined as a desirable signal or an undesirable signal to calculate peak values of the segment signals determined as the desirable signals. Moreover, it is further determined to reserve or discard the undesirable signals and calculate a representative peak value of the reserved undesirable signals.

The processor 110 captures a fixed-length signal by using a sampling window to calculate a peak value here. After the peak value in the sampling window is calculated, a peak value of a signal in the next sampling window is calculated after translating the sampling window back a distance based on the time series. The above translation distance is, for example, 1/2 a width of the sampling window.

When the wearer is stable and has no movement, the physiological signal detected by the heart rate sensor 130 has obvious and regular peak values. When the wearer has a movement, the physiological signal detected by the heart rate sensor 130 has noise. When the physiological signal is mixed with noise, it is difficult to determine a desirable signal, and an intensity of the noise may be higher than the desirable signal, so the intensity cannot be used to determine the desirable signal. The undesirable signal may be defined as having too many peak values here. For example, when the number of the peak values in the segment signal is greater than 1, the segment signal can be determined as the undesirable signal.

In addition, the processor 110 may determine the segment signals as the desirable signals or the undesirable signals by using a dynamic time warping (DTW) algorithm. A principle of the DTW algorithm is to provide a sample waveform and determine whether each waveform to be tested matches the sample waveform. Therefore, a sample waveform of the desirable signal is provided here, so that the waveform of each of the segment signals (that is, the waveform to be tested) is compared with the sample waveform to determine each of the segment signals as the desirable signal or the undesirable signal.

For the i^{th} segment signal determined as the undesirable signal, the processor 110 may further determine a change trend according to the adjacent segment signals in the time series, thereby calculating a representative peak value of the i^{th} segment signal. Thus, it is necessary to firstly determine whether the adjacent segment signals in the time series are reliable, that is, whether they are desirable signals.

Since the physiological signal is a continuous signal, the signal will conform to an overall change trend, so that the peak value close to the overall trend is more likely to be an actual signal, and the peak value that deviates from the overall trend is more likely to be noise. Therefore, in the embodiment, the second segment signal ahead and the second segment signal behind (the (i-2)^{th} segment signal and the (i+2)^{th} segment signal) of the undesirable signal (i^{th} segment signal) are particularly taken out to determine the change trend. The reason for not taking the adjacent previous segment signal and the next segment signal (the (i-1)^{th} segment signal and the (i+1)^{th} segment signal) is that the peak values of the adjacent segment signals are close to the undesirable signal, and may be unstable peak values or be affected by the undesirable signal. Therefore, the peak values of the second segment signal ahead and the second segment signal behind (the (i-2)^{th} segment signal and the (i+2)^{th} segment signal) of the undesirable signal are taken out to further calculate the representative peak value of the undesirable signal. On the one hand, the change trend is still reserved, and on the other hand, it is not easily affected by the undesirable signal.

In the embodiment, the method of calculating the peak value is real-time calculation. Therefore, when the segment signal is determined as the undesirable signal, the segment signal is firstly temporarily stored, and after the peak values of the subsequent two segment signals are taken, it can be determined to reserve or discard the undesirable signal. For the determination to reserve or discard the undesirable signal, reference may be made to step S215 to step S225. In addition, for example, a flag "true" is given to each of the reserved segment signals, and a flag "false" is given to each of the discarded segment signals.

In step S215, if the i^{th} segment signal is determined as the undesirable signal, it is determined whether the (i-2)^{th} segment signal and the (i+2)^{th} segment signal are desirable signals. In step S220, when determining that both of the (i-2)^{th} segment signal and the (i+2)^{th} segment signal are the desirable signals, the i^{th} segment signal is reserved to calculate a representative peak value of the i^{th} segment signal. In step S225, when determining that at least one of the (i-2)^{th} segment signal and the (i+2)^{th} segment signal is the undesirable signal, the i^{th} segment signal is discarded.

For example, assuming that the 3^{rd} segment signal is determined as an undesirable signal, the processor 110 will firstly temporarily store the 3^{rd} segment signal to wait for the obtainment of the 5^{th} segment signal. The processor 110 further determines whether both the 1^{st} segment signal and the 5^{th} whether are the desirable signals. If both of the 1^{st} segment signal and the 5^{th} segment signal are the desirable signals, the 3^{rd} segment signal is reserved, and a representative peak value of the 3^{rd} segment signal is further calculated. If one of the 1^{st} segment signal and the 5^{th} segment signal is the undesirable signal, the 3^{rd} segment signal is discarded, that is, the representative peak value of the 3^{rd} segment signal is not calculated.

Finally, in step S230, instant heart rates are calculated based on the segment signals determined as the desirable signals and the reserved segment signals determined as the undesirable signals. The processor 110 calculates the instant heart rate by using a time difference between the 2 adjacent peak values here, and finally makes statistics on several instant heart rates to obtain an average heart rate.

Generally speaking, the heartbeats per minute F (instant heart rate) is obtained by calculating a time difference T between two adjacent peak values (that is, a heart rate cycle) and then calculating a reciprocal thereof. F = 1/T (heartbeats per second) = 60/T (heartbeats per minute).

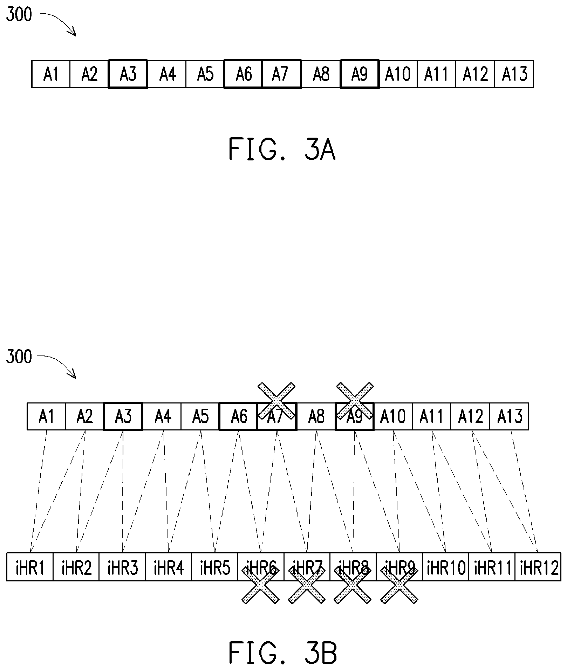

FIG. 3A is a schematic diagram of a physiological signal according to an embodiment of the disclosure. FIG. 3B is a schematic diagram of calculating instant heart rates according to an embodiment of the disclosure. Referring to FIG. 3A, a physiological signal 300 includes a plurality of segment signals, and 13 segment signals A1 to A13 are shown here. The segment signals A1, A2, A4, A5, A8 and A10 to A13 represent desirable signals, and the segment signals A3, A6, A7 and A9 represent undesirable signals. In terms of the segment signal A3, the segment signal A1 is taken ahead and the segment signal A5 is taken behind. Both of the segment signal A1 and the segment signal A5 are the desirable signals, so the segment signal A3 determined as the undesirable signal is reserved. By analogy, the segment signals A3 and A6 are reserved, and the segment signals A7 and A9 are discarded.

Referring to FIG. 3B, the processor 110 obtains instant heart rates based on a time difference of peak values of two adjacent time segments. Based on the above embodiment, the segment signals A3 and A6 determined as the undesirable signals are reserved, so instant heart rates iHR1 to iHR5 may be obtained based on the time segments A1 to A6. Instant heart rates iHR10 to iHR12 may be obtained based on the time segments A10 to A13. In addition, since the time segments A7 and A9 have been discarded, instant heart rates iHR6 to iHR9 that could have been obtained based on the time segments A6 to A10 cannot be obtained. The processor 110 may calculate an average heart rate after obtaining a preset number of instant heart rates here. For example, it may be designed to calculate the average heart rate with 5 instant heart rates. Therefore, through the method of the embodiment, 5 instant heart rates iHR1 to iHR5 may be obtained based on the time segments A1 to A6. Average heart rate = (iHR1 + iHR2 + iHR3 + iHR4 + iHR5)/5.

If the method of the above embodiment is not used, since all of the segment signals A3, A6, A7 and A9 are determined as the undesirable signals, all of the instant heart rates iHR2, iHR3 and iHR5 to iHR9 are unavailable, and it is necessary to delay to the segment signal A13 to obtain 5 instant heart rates iHR1, iHR4, iHR10, iHR11 and iHR12. Therefore, through the method of the above embodiment, the problem of delay in the calculation of the average heart rate can be solved.

In addition, in the above step S220, the representative peak value of the i^{th} segment signal may be calculated based on a first peak value of the (i-2)^{th} segment signal and a second peak value of the (i+2)^{th} segment signal, and may be selected from a plurality of candidate peak values included in the i^{th} segment signal. Specifically, the processor 110 determines a change trend of the i^{th} segment signal based on the first peak value of the (i-2)^{th} segment signal and the second peak value of the (i+2)^{th} segment signal. Then, the processor 110 obtains the representative peak value of the i^{th} segment signal by using a calculation manner corresponding to the change trend.

The i^{th} segment signal determined as the undesirable signal has three change trends, including a stable state, a continuous increase state and a continuous decrease state. The processor 110 calculates an absolute value of a difference between the first peak value and the second peak value (|first peak value-second peak value|), and determines the change trend of the i^{th} segment signal based on the absolute value of the difference. The processor 110 compares the absolute value of the difference with a preset threshold value here. If the absolute value of the difference is less than or equal to the threshold value, it is determined that the change trend of the i^{th} segment signal is the stable state. If the absolute value of the difference is greater than the threshold value and the first peak value is less than the second peak value, it is determined that the change trend of the i^{th} segment signal is the continuous increase state. If the absolute value of the difference is greater than the threshold value and the first peak value is greater than the second peak value, it is determined that the change trend of the i^{th} segment signal is the continuous decrease state.

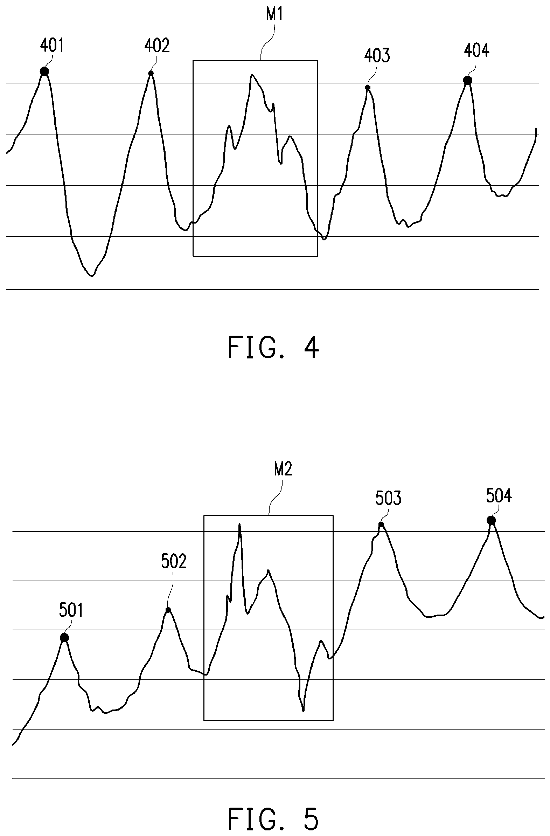

The stable state, the continuous increase state and the continuous decrease state are respectively described below with reference to FIG. 4 to FIG. 6.

FIG. 4 is a waveform diagram of a stable state according to an embodiment of the disclosure. In FIG. 4, it is assumed that the m^{th} segment signal to the (m+4)^{th} segment signal are shown. The (m+2)^{th} segment signal (segment signal M1) is an undesirable signal, and the other segment signals are desirable signals. Peak values 401, 402, 403 and 404 are respectively peak values of the m^{th} segment signal, the (m+1)^{th} segment signal, the (m+3)^{th} segment signal and the (m+4)^{th} segment signal.

In terms of the segment signal M1, the processor 110 takes the peak value 401 (first peak value) of the m^{th} segment signal ahead, takes the peak value 404 (second peak value) of the (m+4)^{th} segment signal behind, calculates an absolute value of a difference between the peak value 401 and the peak value 404, and compares the absolute value of the difference with a preset threshold value. Since the absolute value of the difference between the peak value 401 and the peak value 404 is not greater than the threshold value, it is determined that the change trend of the segment signal M1 is the stable state. It is assumed that the preset threshold value is 1500 millivolts (mV) here.

FIG. 5 is a waveform diagram of a continuous increase state according to an embodiment of the disclosure. In FIG. 5, it is assumed that the n^{th} segment signal to the (n+4)^{th} segment signal are shown. The (n+2)^{th} segment signal (segment signal M2) is an undesirable signal, and the other segment signals are desirable signals. Peak values 501, 502, 503 and 504 are respectively peak values of the n^{th} segment signal, the (n+1)^{th} segment signal, the (n+3)^{th} segment signal and the (n+4)^{th} segment signal.

In terms of the segment signal M2, the processor 110 takes the peak value 501 (first peak value) of the n^{th} segment signal ahead, takes the peak value 504 (second peak value) of the (n+4)^{th} segment signal behind, calculates an absolute value of a difference between the peak value 501 and the peak value 504, and compares the absolute value of the difference with a preset threshold value. Since the absolute value of the difference between the peak value 501 and the peak value 504 is greater than the threshold value, it is further determined whether the peak value 501 or the peak value 504 is greater. The peak value 504 is greater than the peak value 501 here, so it is determined that the change trend of the segment signal M2 is the continuous increase state.

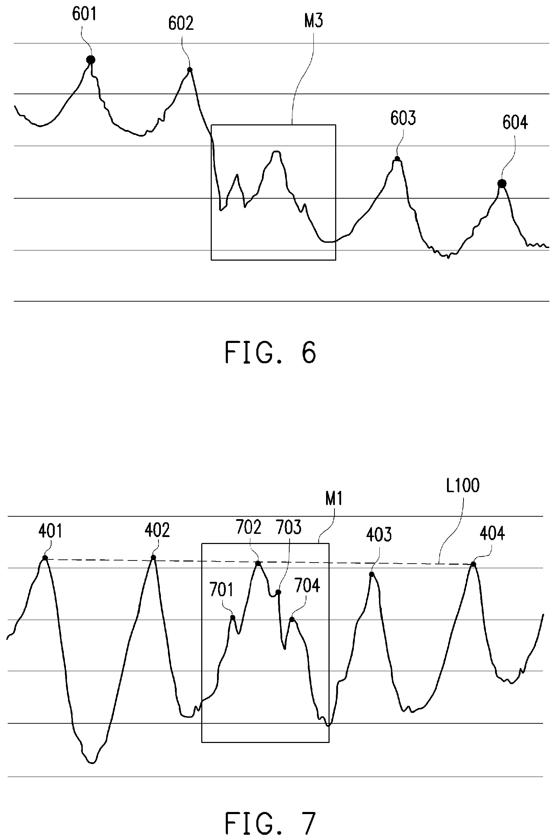

FIG. 6 is a waveform diagram of a continuous decrease state according to an embodiment of the disclosure. In FIG. 6, it is assumed that the p^{th} segment signal to the (p+4)^{th} segment signal are shown. The (p+2)^{th} segment signal (segment signal M3) is an undesirable signal, and the other segment signals are desirable signals. Peak values 601, 602, 603 and 604 are respectively peak values of the p^{th} segment signal, the (p+1)^{th} segment signal, the (p+3)^{th} segment signal and the (p+4)^{th} segment signal.

In terms of the segment signal M3, the processor 110 takes the peak value 601 (first peak value) of the p^{th} segment signal ahead, takes the peak value 604 (second peak value) of the (p+4)^{th} segment signal behind, calculates an absolute value of a difference between the peak value 601 and the peak value 604, and compares the absolute value of the difference with a preset threshold value. Since the absolute value of the difference between the peak value 601 and the peak value 604 is greater than the threshold value, it is further determined whether the peak value 601 or the peak value 604 is greater. The peak value 601 is greater than the peak value 604 here, so it is determined that the change trend of the segment signal M3 is the continuous decrease state.

After obtaining the change trend of the undesirable signal, the processor 110 may obtain the representative peak value of the undesirable signal by using the calculation manner corresponding to the change trend. The calculation manners of the representative peak values in the stable state, the continuous increase state and the continuous decrease state are respectively described below with reference to FIG. 7, FIG. 8A to FIG. 8B and FIG. 9A to FIG. 9B.

FIG. 7 is a waveform diagram of a calculation manner of a representative peak value in a stable state according to an embodiment of the disclosure. Here, the embodiment of FIG. 4 is used to describe the calculation manner of the representative peak value in the stable state.

Referring to FIG. 7, firstly, the processor 110 obtains a line segment L100 based on the peak value 401 (first peak value) and the peak value 404 (second peak value). Next, the processor 110 finds out a plurality of candidate peak values 701 to 704 from the segment signal M1, and takes out one of the candidate peak values closest to the line segment L100 from these candidate peak values 701 to 704 as a representative peak value of the segment signal M1. The candidate peak value 702 is selected as the representative peak value of the segment signal M1.

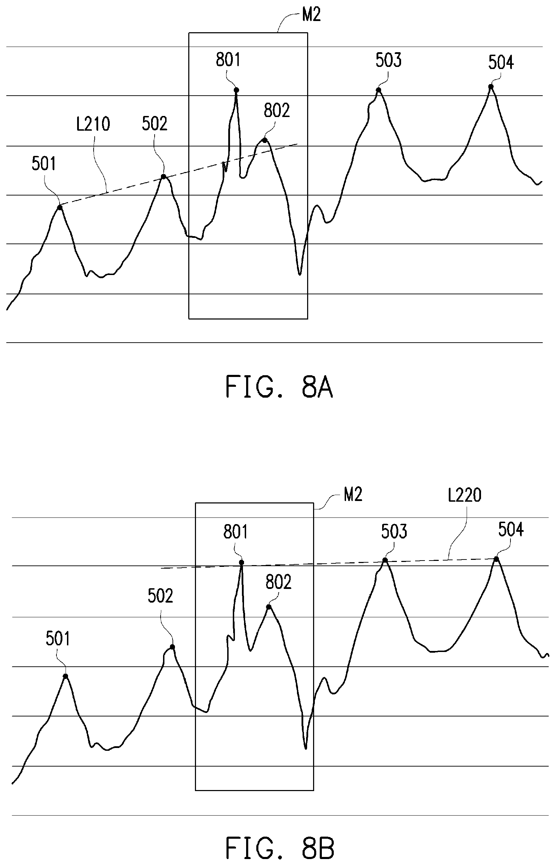

FIG. 8A to FIG. 8B are waveform diagrams of a calculation manner of a representative peak value in a continuous increase state according to an embodiment of the disclosure. The embodiment of FIG. 5 is used to describe the calculation manner of the representative peak value in the continuous increase state here.

Referring to FIG. 8A to FIG. 8B, the (n+2)^{th} segment signal (segment signal M2) is the undesirable signal. The processor 110 obtains a line segment L210 based on the peak value 501 (first peak value) of the n^{th} segment signal and the peak value 502 (third peak value) of the (n+1)^{th} segment signal. Moreover, the processor 110 obtains a line segment L220 based on the peak value 504 (second peak value) of the (n+4)^{th} segment signal and the peak value 503 (fourth peak value) of the (n+3)^{th} segment signal.

Moreover, the processor 110 finds out a plurality of candidate peak values 801 and 802 from the segment signal M2. Next, the processor 110 takes out the candidate peak value 802 (first candidate peak value) closest to the line segment L210 from these candidate peak values 801 and 802, and takes out the candidate peak value 801 (second candidate peak value) closest to the line segment L220 from these candidate peak values 801 and 802. Next, the processor 110 determines whether the first candidate peak value and the second candidate peak value are the same.

In the waveform where the change trend is the continuous increase state, since a surge with a sudden increase in intensity is more likely to be noise, if the first candidate peak value and the second candidate peak value are different, the smallest of the first candidate peak value and the second candidate peak value is taken as the representative peak value of the segment signal M2. In the embodiment of FIG. 8A to FIG. 8B, the first candidate peak value (candidate peak value 802) and the second candidate peak value (candidate peak value 801) are different, so the processor 110 selects the smallest candidate peak value 802 of the two as the representative peak value of the segment signal M2.

In other embodiments, if the first candidate peak value closest to the line segment L210 and the second candidate peak value closest to the line segment L220 are the same, the processor 110 takes the first candidate peak value (that is, the second candidate peak value) as the representative peak value of the segment signal M2.

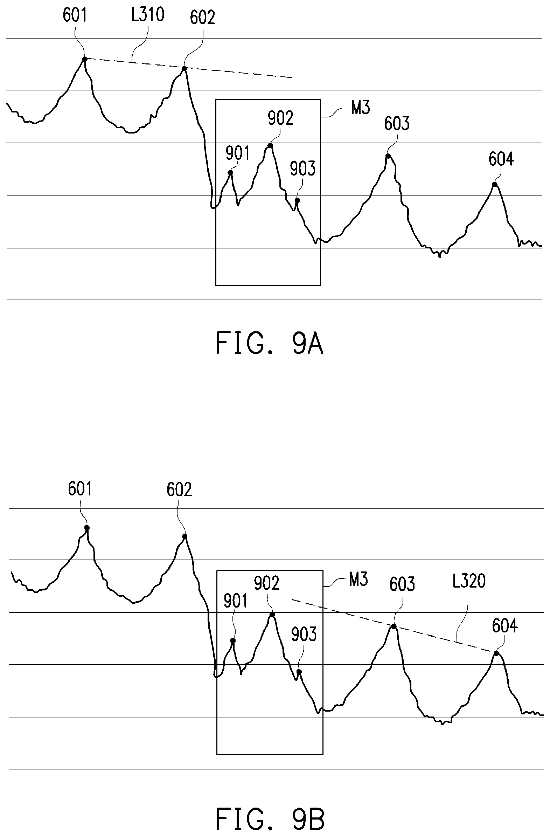

FIG. 9A to FIG. 9B are waveform diagrams of a calculation manner of a representative peak value in a continuous decrease state according to an embodiment of the disclosure. The embodiment of FIG. 6 is used to describe the calculation manner of the representative peak value in the continuous decrease state here.

Referring to FIG. 9A to FIG. 9B, the (p+2)^{th} segment signal (segment signal M3) is the undesirable signal. The processor 110 obtains a line segment L310 based on the peak value 601 (first peak value) of the p^{th} segment signal and the peak value 602 (third peak value) of the (p+1)^{th} segment signal. Moreover, the processor 110 obtains a line segment L320 based on the peak value 604 (second peak value) of the (p+4)^{th} segment signal and the peak value 603 (fourth peak value) of the (p+3)^{th} segment signal.

Moreover, the processor 110 finds out a plurality of candidate peak values 901, 902 and 903 from the segment signal M3. Next, the processor 110 takes out the candidate peak value 902 (first candidate peak value) closest to the line segment L310 from these candidate peak values 901, 902 and 903, and takes out the candidate peak value 902 (second candidate peak value) closest to the line segment L320 from these candidate peak values 901, 902 and 903. Next, the processor 110 determines whether the first candidate peak value and the second candidate peak value are the same.

In the embodiment, both of the first candidate peak value and the second candidate peak value are the candidate peak value 902, so the processor 110 takes the candidate peak value 902 as the representative peak value of the segment signal M3.

In other embodiments, in the waveform where the change trend is the continuous decrease state, since a surge with a sudden decrease in intensity is more likely to be noise, if the first candidate peak value closest to the line segment L310 and the second candidate peak value closest to the line segment L320 are different, the largest of the first candidate peak value and the second candidate peak value is taken as the representative peak value of the segment signal M3.

In addition, in the above embodiment, the representative peak value of the undesirable signal is further calculated. Therefore, in order to avoid a too absurd determination result, after obtaining the representative peak value of the undesirable signal, it is further calculated whether a voltage value of the representative peak value of the undesirable signal is too high. For example, the i^{th} segment signal is determined as the undesirable signal, and a comparison value is obtained based on an average value of a first peak value of the (i-2)^{th} segment signal and a second peak value of the (i+2)^{th} segment signal. Comparison value = 2 × ACAVG(S1AC:S2AC). ACAVG(S1AC:S2AC) represents an average value of a voltage value S1AC of the first peak value of the (i-2)^{th} segment signal and a voltage value S2AC of the second peak value of the (i+2)^{th} segment signal.

Next, the representative peak value of the undesirable signal is compared with the comparison value. If the representative peak value is greater than the comparison value, it is determined that the undesirable signal is unreliable, and the undesirable signal is discarded. If the representative peak value is less than or equal to the comparison value, it is determined that the undesirable signal is reliable, and the undesirable signal is reserved.

In addition, the above embodiments may be realized by a non-transitory computer readable storage medium. The non-transitory computer readable storage medium records at least one programmed instruction. After being loaded into an electronic device, the programmed instruction performs steps S205 to S230.

Based on the above, in the disclosure, for the segment signal of undesirable quality, the possible representative peak value is calculated based on the change trend of the signals ahead and behind, the peak values are found out from the PPG signal of undesirable quality, the problem of delay in the average heart rate caused by the undesirable quality of the physiological signal is solved, and the average heart rate can be output stably.

### [REFERENCE SIGNS LIST]

100: signal processing apparatus
110: processor
120: storage unit
130: heart rate sensor
300: physiological signal
401-404, 501-504, 601-604: peak value
701-704, 801, 802, 901-903: candidate peak value
A1-A13 , M1-M3: segment signal
iHR1-iHR12: instant heart rate
L100, L210, L220, L310, L320: line segment
S205-S230: steps of signal processing method

## Claims

1. A signal processing method, suitable for an electronic device (100) including a processor (110), wherein the signal processing method comprises following steps:
dividing, by the processor (110), a physiological signal (300) into a plurality of segment signals (A1-A13) based on a time series;
taking out, by the processor (110), a i^{th} segment signal from the plurality of segment signals (A1-A13) one by one according to the time series, and determining, by the processor (110), whether the i^{th} segment signal is a desirable signal or an undesirable signal;
in response to the i^{th} segment signal (A1, A2, A4, A5, A8, A10-A13) being determined as the desirable signal, reserving, by the processor (110), the i^{th} segment signal, and calculating a peak value of the reserved i^{th} segment signal determined as the desirable signal;
in response to the i^{th} segment signal (A3, A6, A7, A9) being determined as the undesirable signal, performing a confirmation step by the processor (110), wherein the confirmation step comprises:
determining whether a (i-2)^{th} segment signal and a (i+2)^{th} segment signal are the desirable signals;
under the condition of determining that both of the (i-2)^{th} segment signal and the (i+2)^{th} segment signal are the desirable signals, reserving the i^{th} segment signal (A3, A6, M1-M3) determined as the undesirable signal, and calculating a representative peak value (702, 802, 902) based on the (i-2)^{th} segment signal and the (i+2)^{th} segment signal, wherein the representative peak value (702, 802, 902) represents the peak value corresponding to the reserved i^{th} segment signal (A3, A6, M1-M3) determined as the undesirable signal; and
under the condition of determining that at least one of the (i-2)^{th} segment signal and the (i+2)^{th} segment signal is the undesirable signal, discarding the i^{th} segment signal (A7, A9) determined as the undesirable signal; and
calculating, by the processor (110), a plurality of instant heart rates (iHR1-iHR12) based on a plurality of peak values (401-404, 501-504, 601-604, 702, 802, 902) of the reserved segment signals.

2. The signal processing method according to claim 1, wherein the step of calculating the representative peak value (702, 802, 902) based on the (i-2)^{th} segment signal and the (i+2)^{th} segment signal comprises following steps :
determining, by the processor (110), a change trend of the i^{th} segment signal (M1-M3) based on a first peak value (401, 501, 601) of the (i-2)^{th} segment signal and a second peak value (404, 504, 604) of the (i+2)^{th} segment signal; and
obtaining, by the processor (110), the representative peak value (702, 802, 902) of the i^{th} segment signal (M1-M3) by using a calculation manner corresponding to the change trend.

3. The signal processing method according to claim 2, wherein before the step of determining, by the processor (110), the change trend of the i^{th} segment signal (M1-M3), the signal processing method further comprises following steps:
calculating, by the processor (110), an absolute value of a difference between the first peak value (401, 501, 601) and the second peak value (404, 504, 604);
wherein the step of determining, by the processor (110), the change trend of the i^{th} segment signal (M1-M3) comprises:
comparing the absolute value of the difference with a threshold value;
in response to the absolute value of the difference being less than or equal to the threshold value, determining, by the processor (110), that the change trend of the i^{th} segment signal (M1) is a stable state;
in response to the absolute value of the difference being greater than the threshold value and the first peak value (501) is less than the second peak value (504), determining, by the processor (110), that the change trend of the i^{th} segment signal (M2) is a continuous increase state; and
in response to the absolute value of the difference being greater than the threshold value and the first peak value (601) is greater than the second peak value (604), determining, by the processor (110), that the change trend of the i^{th} segment signal (M3) is a continuous decrease state.

4. The signal processing method according to claim 3, wherein the step of obtaining, by the processor (110), the representative peak value (702, 802, 902) of the i^{th} segment signal (M1-M3) by using the calculation manner corresponding to the change trend comprises:
under the condition that the change trend is the stable state,
obtaining, by the processor (110), a line segment (L100) based on the first peak value (401) and the second peak value (404);
finding out, by the processor (110), a plurality of candidate peak values (701-704) from the i^{th} segment signal (M1); and
taking out, by the processor (110), one of the candidate peak values (701-704) closest to the line segment (L100) from the plurality of candidate peak values (701-704) as the representative peak value (702) of the i^{th} segment signal (M1).

5. The signal processing method according to claim 3, wherein the step of obtaining, by the processor (110), the representative peak value (702, 802, 902) of the i^{th} segment signal (M1-M3) by using the calculation manner corresponding to the change trend comprises:
under the condition that the change trend is the continuous increase state,
obtaining, by the processor (110), a first line segment (L210) based on the first peak value (501) and a third peak value (502) of a (i-1)^{th} segment signal;
obtaining, by the processor (110), a second line segment (L220) based on the second peak value (504) and a fourth peak value (503) of a (i+1)^{th} segment signal;
finding out, by the processor (110), a plurality of candidate peak values (801, 802) from the i^{th} segment signal (M2);
taking out, by the processor (110), a first candidate peak value (802) closest to the first line segment (L210) from the plurality of candidate peak values (801, 802);
taking out, by the processor (110), a second candidate peak value (801) closest to the second line segment (L220) from the plurality of candidate peak values (801, 802);
in response to the first candidate peak value (802) and the second candidate peak value (801) being the same, taking, by the processor (110), the first candidate peak value (802) as the representative peak value (802) of the i^{th} segment signal (M2); and
in response to the first candidate peak value (802) and the second candidate peak value (801) being different, taking, by the processor (110), the smallest of the first candidate peak value (802) and the second candidate peak value (801) as the representative peak value (802) of the i^{th} segment signal (M2).

6. The signal processing method according to claim 3, wherein the step of obtaining, by the processor (110), the representative peak value (702, 802, 902) of the i^{th} segment signal (M1-M3) by using the calculation manner corresponding to the change trend comprises:
under the condition that the change trend is the continuous decrease state,
obtaining, by the processor (110), a first line segment (L310) based on the first peak value (601) and a third peak value (602) of a (i-1)^{th} segment signal;
obtaining, by the processor (110), a second line segment (L320) based on the second peak value (604) and a fourth peak value (603) of a (i+1)^{th} segment signal;
finding out, by the processor (110), a plurality of candidate peak values (901-903) from the i^{th} segment signal (M3);
taking out, by the processor (110), a first candidate peak value (902) closest to the first line segment (L310) from the plurality of candidate peak values (901-903);
taking out, by the processor (110), a second candidate peak value (902) closest to the second line segment (L320) from the plurality of candidate peak values (901-903);
in response to the first candidate peak value (902) and the second candidate peak value (902) being the same, taking, by the processor (110), the first candidate peak value (902) as the representative peak value (902) of the i^{th} segment signal (M3); and
in response to the first candidate peak value and the second candidate peak value being different, taking, by the processor (110), the largest of the first candidate peak value and the second candidate peak value as the representative peak value of the i^{th} segment signal (M3).

7. The signal processing method according to one of claims 1 to 6, wherein the step of determining, by the processor (110), whether the i^{th} segment signal is the desirable signal or the undesirable signal comprises:
determining, by the processor (110), whether the i^{th} segment signal is the desirable signal or the undesirable signal by using a dynamic time warping algorithm.

8. The signal processing method according to one of claims 1 to 7, wherein after reserving the i^{th} segment signal (A3, A6, M1-M3) determined as the undesirable signal, and calculating the representative peak value (702, 802, 902) based on the (i-2)^{th} segment signal and the (i+2)^{th} segment signal, the signal processing method further comprises following steps:
obtaining, by the processor (110), a comparison value based on an average value of a first peak value of the (i-2)^{th} segment signal and a second peak value of the (i+2)^{th} segment signal;
determining, by the processor (110), whether the representative peak value (702, 802, 902) is greater than the comparison value;
in response to the representative peak value (702, 802, 902) being greater than the comparison value, determining, by the processor (110), that the i^{th} segment signal (M1-M3) is unreliable, and discarding the i^{th} segment signal (M1-M3); and
in response to the representative peak value (702, 802, 902) being less than or equal to the comparison value, determining that the i^{th} segment signal (M1-M3) is reliable, and reserving the i^{th} segment signal (M1-M3).

9. A signal processing apparatus (100), comprising:
a heart rate sensor (130), configured to sense a physiological signal (300) of a wearer;
a storage unit (120), configured to comprise a plurality of code segments; and
a processor (110), configured to be coupled to the heart rate sensor (130) and the storage unit (120), wherein the processor (110) is configured to execute the plurality of code segments to:
receive the physiological signal (300) from the heart rate sensor (130);
divide the physiological signal (300) into a plurality of segment signals (A1-A13) based on a time series;
take out a i^{th} segment signal from the plurality of segment signals (A1-A13) one by one according to the time series, and determine whether the i^{th} segment signal is a desirable signal or an undesirable signal;
in response to the i^{th} segment signal (A1, A2, A4, A5, A8, A10-A13) being determined as the desirable signal, reserve the i^{th} segment signal, and calculate a peak value of the reserved i^{th} segment signal determined as the desirable signal;
in response to the i^{th} segment signal (A3, A6, A7, A9) being determined as the undesirable signal, perform a confirmation step by the processor (110), wherein the confirmation step comprises:
determine whether a (i-2)^{th} segment signal and a (i+2)^{th} segment signal are the desirable signals;
under the condition of determining that both of the (i-2)^{th} segment signal and the (i+2)^{th} segment signal are the desirable signals, reserve the i^{th} segment signal (A3, A6, M1-M3) determined as the undesirable signal, and calculate a representative peak value (702, 802, 902) based on the (i-2)^{th} segment signal and the (i+2)^{th} segment signal, wherein the representative peak value (702, 802, 902) represents the peak value corresponding to the reserved i^{th} segment signal (A3, A6, M1-M3) determined as the undesirable signal; and
under the condition of determining that at least one of the (i-2)^{th} segment signal and the (i+2)^{th} segment signal is the undesirable signal, discarding the i^{th} segment signal (A7, A8) determined as the undesirable signal; and
calculate a plurality of instant heart rates (iHR1-iHR12) based on a plurality of peak values (401-404, 501-504, 601-604, 702, 802, 902) of the reserved segment signals.

10. The signal processing apparatus (100) according to claim 9, wherein the processor (110) is configured to execute the plurality of code segments to:
determine a change trend of the i^{th} segment signal (M1-M3) based on a first peak value (401, 501, 601) of the (i-2)^{th} segment signal and a second peak value (404, 504, 604) of the (i+2)^{th} segment signal; and
obtain the representative peak value (702, 802, 902) of the i^{th} segment signal (M1-M3) by using a calculation manner corresponding to the change trend.

11. The signal processing apparatus (100) according to claim 10, wherein the processor (110) is configured to execute the plurality of code segments to:
before determining the change trend of the i^{th} segment signal (M1-M3), calculate an absolute value of a difference between a first peak value (401, 501, 601) and a second peak value (404, 504, 604);
compare the absolute value of the difference with a threshold value, thereby determining the change trend of the i^{th} segment signal (M1-M3);
in response to the absolute value of the difference being less than or equal to the threshold value, determine that the change trend of the i^{th} segment signal (M1) is a stable state;
in response to the absolute value of the difference being greater than the threshold value and the first peak value (501) is less than the second peak value (504), determine that the change trend of the i^{th} segment signal (M2) is a continuous increase state; and
in response to the absolute value of the difference being greater than the threshold value and the first peak value (601) is greater than the second peak value (604), determine that the change trend of the i^{th} segment signal (M3) is a continuous decrease state,
under the condition that the change trend is the stable state,
obtain a line segment (L100) based on the first peak value (401) and the second peak value (404);
find out a plurality of candidate peak values (701-704) from the i^{th} segment signal (M1); and
take out one of the candidate peak values (701-704) closest to the line segment (L100) from the plurality of candidate peak values (701-704) as the representative peak value (702) of the i^{th} segment signal (M1),
under the condition that the change trend is the continuous increase state,
obtain a first line segment (L210) based on the first peak value (501) and a third peak value (502) of a (i-1)^{th} segment signal;
obtain a second line segment (L220) based on the second peak value (504) and a fourth peak value (503) of a (i+1)^{th} segment signal;
find out a plurality of candidate peak values (801, 802) from the i^{th} segment signal (M2);
take out a first candidate peak value (802) closest to the first line segment (L210) from the plurality of candidate peak values (801, 802);
take out a second candidate peak value (802) closest to the second line segment (L220) from the plurality of candidate peak values (801, 802);
in response to the first candidate peak value (802) and the second candidate peak value (801) being the same, take the first candidate peak value (802) as the representative peak value (802) of the i^{th} segment signal (M2); and
in response to the first candidate peak value (802) and the second candidate peak value (801) being different, take the smallest of the first candidate peak value (802) and the second candidate peak value (801) as the representative peak value (802) of the i^{th} segment signal (M2),
under the condition that the change trend is the continuous decrease state,
obtain a first line segment (L310) based on the first peak value (601) and a third peak value (602) of a (i-1)^{th} segment signal;
obtain a second line segment (L320) based on the second peak value (604) and a fourth peak value (603) of a (i+1)^{th} segment signal;
find out a plurality of candidate peak values (901-903) from the i^{th} segment signal (M3);
take out a first candidate peak value (902) closest to the first line segment (L310) from the plurality of candidate peak values (901-903);
take out a second candidate peak value (902) closest to the second line segment (L320) from the plurality of candidate peak values (901-903);
in response to the first candidate peak value (902) and the second candidate peak value (902) being the same, take the first candidate peak value (902) as the representative peak value (902) of the i^{th} segment signal (M3); and
in response to the first candidate peak value and the second candidate peak value being different, take the largest of the first candidate peak value and the second candidate peak value as the representative peak value of the i^{th} segment signal (M3).

12. The signal processing apparatus (100) according to one of claims 9 to 11, wherein the processor (110) executes the plurality of code segments to:
after reserving the i^{th} segment signal (A3, A6, M1-M3) determined as the undesirable signal, and calculating the representative peak value (702, 802, 902) based on the (i-2)^{th} segment signal and the (i+2)^{th} segment signal, obtain a comparison value based on an average value of a first peak value of the (i-2)^{th} segment signal and a second peak value of the (i+2)^{th} segment signal;
determine whether the representative peak value (702, 802, 902) is greater than the comparison value;
in response to the representative peak value (702, 802, 902) being greater than the comparison value, determine that the i^{th} segment signal (M1-M3) is unreliable, and discard the i^{th} segment signal (M1-M3); and
in response to the representative peak value (702, 802, 902) being less than or equal to the comparison value, determine that the i^{th} segment signal (M1-M3) is reliable, and reserve the i^{th} segment signal (M1-M3).

13. A non-transitory computer readable storage medium, wherein the non-transitory computer readable storage medium records at least one programmed instruction, and after the at least one programmed instruction is loaded into an electronic device, a processor (110) of the electronic device performs following steps:
dividing a physiological signal (300) into a plurality of segment signals (A1-A13) based on a time series;
taking out a i^{th} segment signal from the plurality of segment signals (A1-A13) one by one according to the time series, and determining whether the i^{th} segment signal is a desirable signal or an undesirable signal;
in response to the i^{th} segment signal (A1, A2, A4, A8, A10-A13) being determined as the desirable signal, reserving the i^{th} segment signal, and calculating a peak value of the reserved i^{th} segment signal determined as the desirable signal;
in response to the i^{th} segment signal (A3, A6, A7, A8) being determined as the undesirable signal, performing a confirmation step, wherein the confirmation step comprises:
determining whether a (i-2)^{th} segment signal and a (i+2)^{th} segment signal are the desirable signals;
under the condition of determining that both of the (i-2)^{th} segment signal and the (i+2)^{th} segment signal are the desirable signals, reserving the i^{th} segment signal (A3, A6, M1-M3) determined as the undesirable signal, and calculating a representative peak value (702, 802, 902) based on the (i-2)^{th} segment signal and the (i+2)^{th} segment signal, wherein the representative peak value (702, 802, 902) represents the peak value corresponding to the reserved i^{th} segment signal (A3, A6, M1-M3) determined as the undesirable signal; and
under the condition of determining that at least one of the (i-2)^{th} segment signal and the (i+2)^{th} segment signal is the undesirable signal, discarding the i^{th} segment signal (A7, A8) determined as the undesirable signal; and
calculating a plurality of instant heart rates (iHR1-iHR12) based on a plurality of peak values (401-404, 501-504, 601-604, 702, 802, 902) of the reserved segment signals.

## Patentansprüche

1. Ein Signalverarbeitungsverfahren, das für ein elektronisches Gerät (100) mit einem Prozessor (110) geeignet ist, wobei das Signalverarbeitungsverfahren die folgenden Schritte umfasst:
Aufteilen eines physiologischen Signals (300) durch den Prozessor (110) in eine Vielzahl von Segmentsignalen (A1-A13) auf der Grundlage einer Zeitreihe;
Entnehmen eines i^{-ten} Segmentsignals aus der Vielzahl von Segmentsignalen (A1-A13) nacheinander gemäß der Zeitreihe durch den Prozessor (110) und Bestimmen, ob das i^{-te} Segmentsignal ein gewünschtes Signal oder ein unerwünschtes Signal ist, durch den Prozessor (110);
als Reaktion darauf, dass das i^{-te} Segmentsignal (A1, A2, A4, A5, A8, A10-A13) als das wünschenswerte Signal bestimmt wurde, Reservieren des i^{-ten} Segmentsignals durch den Prozessor (110) und Berechnen eines Spitzenwerts des reservierten i^{-ten} Segmentsignals, das als das wünschenswerte Signal bestimmt wurde;
als Reaktion darauf, dass das i^{-te}Segmentsignal (A3, A6, A7, A9) als unerwünschtes Signal bestimmt wurde, Durchführen eines Bestätigungsschritts durch den Prozessor (110), wobei der Bestätigungsschritt umfasst:
Bestimmen, ob ein (i-2)-^{tes} Segmentsignal und ein (i+2)-^{tes} Segmentsignal die gewünschten Signale sind;
unter der Bedingung, dass sowohl das (i-2)^{-te} Segmentsignal als auch das (i+2^{)-te} Segmentsignal als wünschenswerte Signale bestimmt werden, das als unerwünschtes Signal bestimmte i^{-te} Segmentsignal (A3, A6, M1-M3) reserviert wird und ein repräsentativer Spitzenwert (702, 802, 902) auf der Grundlage des (i-2)-^{ten}-Segmentsignals und des (i+2)-^{ten} Segmentsignals berechnet wird, wobei der repräsentative Spitzenwert (702, 802, 902) den Spitzenwert darstellt, der dem zurückbehaltenen i-^{ten} Segmentsignal (A3, A6, M1-M3) entspricht, das als unerwünschtes Signal bestimmt wurde; und
unter der Bedingung, dass mindestens eines der (i-2)-^{ten}-Segmentsignale und der (i+2)-^{ten}-Segmentsignale als unerwünschtes Signal bestimmt wird, Verwerfen des i-Segmentsignals(A7, A9), das als unerwünschtes Signal bestimmt wurde; und
Berechnen einer Vielzahl von instanten Herzraten (iHR1-iHR12) durch den Prozessor (110) auf der Grundlage einer Vielzahl von Spitzenwerten (401-404, 501-504, 601-604, 702, 802, 902) der reservierten Segmentsignale.

2. Das Signalverarbeitungsverfahren gemäß Anspruch 1, wobei der Schritt des Berechnens des repräsentativen Spitzenwerts (702, 802, 902) auf der Grundlage des (i-2)^{-ten} Segmentsignals und des (i+2)^{-ten} Segmentsignals die folgenden Schritte umfasst:
Bestimmen einer Änderungsrate des i-^{ten} Segmentsignals (M1-M3) durch den Prozessor (110) auf der Grundlage eines ersten Spitzenwerts (401, 501, 601) des (i-2)-^{ten}-Segmentsignals und eines zweiten Spitzenwerts (404, 504, 604) des (i+2)^{-ten}-Segmentsignals; und
Ermitteln des repräsentativen Spitzenwerts (702, 802, 902) des i^{-ten} Segmentsignals (M1-M3) durch den Prozessor (110) unter Verwendung einer dem Änderungstrend entsprechenden Berechnungsmethode.

3. Das Signalverarbeitungsverfahren gemäß Anspruch 2, wobei vor dem Schritt des Bestimmens des Änderungstrends des i^{ten} Segmentsignals (M1-M3) durch den Prozessor (110) das Signalverarbeitungsverfahren ferner die folgenden Schritte umfasst:
Berechnen eines Absolutwerts einer Differenz zwischen dem ersten Spitzenwert (401, 501, 601) und dem zweiten Spitzenwert (404, 504, 604) durch den Prozessor (110);
wobei der Schritt des Bestimmens der Änderung des i^{ten} Segmentsignale(M1-M3) durch den Prozessor (110) umfasst:
Vergleichen des Absolutwerts der Differenz mit einem Schwellenwert;
als Reaktion darauf, dass der Absolutwert der Differenz kleiner oder gleich dem Schwellenwert ist, Bestimmen durch den Prozessor (110), dass der Änderungstrend des i^{-ten} Segmentsignals (M1) ein stabiler Zustand ist;
als Reaktion darauf, dass der Absolutwert der Differenz größer als der Schwellenwert ist und der erste Spitzenwert (501) kleiner als der zweite Spitzenwert (504) ist, Bestimmen durch den Prozessor (110), dass der Veränderungstrend des i^{-ten} Segmentsignals (M2) ein kontinuierlicher Anstiegszustand ist; und
als Reaktion darauf, dass der Absolutwert der Differenz größer als der Schwellenwert ist und der erste Spitzenwert (601) größer als der zweite Spitzenwert (604) ist, durch den Prozessor (110) bestimmen, dass der Änderungstrend des i^{-ten} Segmentsignals(M3) ein kontinuierlicher Abnahmezustand ist.

4. Das Signalverarbeitungsverfahren gemäß Anspruch 3, wobei der Schritt des Erhaltens des repräsentativen Spitzenwerts (702, 802, 902) des i^{-ten} Segmentsignals (M1-M3) durch den Prozessor (110) unter Verwendung der dem Änderungstrend entsprechenden Berechnungsweise umfasst:
unter der Bedingung, dass der Veränderungstrend der stabile Zustand ist,
Erhalten eines Liniensegments (L100) durch den Prozessor (110) auf der Grundlage des ersten Spitzenwerts (401) und des zweiten Spitzenwerts (404);
Ermitteln einer Vielzahl von Kandidaten-Spitzenwerten (701-704) aus dem ^{i-ten} Segmentsignal (M1) durch den Prozessor (110); und
Auswählen eines der Kandidaten-Spitzenwerte (701-704), der dem Liniensegment (L100) am nächsten liegt, aus der Vielzahl von Kandidaten-Spitzenwerten (701-704) als repräsentativen Spitzenwert (702) des i^{-ten} Segmentsignals (M1) durch den Prozessor (110).

5. Das Signalverarbeitungsverfahren gemäß Anspruch 3, wobei der Schritt des Erhaltens des repräsentativen Spitzenwerts (702, 802, 902) des i-^{ten} Segmentsignals (M1-M3) durch den Prozessor (110) unter Verwendung der dem Änderungstrend entsprechenden Berechnungsweise umfasst:
unter der Bedingung, dass der Änderungstrend der Zustand eines kontinuierlichen Anstiegs ist,
Erhalten eines ersten Liniensegments (L210) durch den Prozessor (110) auf der Grundlage des ersten Spitzenwerts (501) und eines dritten Spitzenwerts (502) eines (i-1)^{-ten} Segmentsignals;
Erhalten eines zweiten Liniensegments (L220) durch den Prozessor (110) auf der Grundlage des zweiten Spitzenwerts (504) und eines vierten Spitzenwerts (503) eines (i+1)^{-ten} Segmentsignals;
Ermitteln einer Vielzahl von Kandidaten-Spitzenwerten (801, 802) aus dem i^{ten} Segmentsignal (M2) durch den Prozessor (110);
Herausnehmen eines ersten Kandidatenspitzenwerts (802), der dem ersten Liniensegment (L210) am nächsten liegt, aus der Vielzahl von Kandidatenspitzenwerten (801, 802) durch den Prozessor (110);
Auswählen eines zweiten Kandidatenspitzenwerts (801), der dem zweiten Liniensegment (L220) am nächsten liegt, aus der Vielzahl von Kandidatenspitzenwerten (801, 802) durch den Prozessor (110);
als Reaktion darauf, dass der erste Kandidatenspitzenwert (802) und der zweite Kandidatenspitzenwert (801) identisch sind, den ersten Kandidatenspitzenwert (802) als repräsentativen Spitzenwert (802) des i^{-ten} Segmentsignals(M2) durch den Prozessor (110) zu nehmen; und
als Reaktion darauf, dass der erste Kandidatenspitzenwert (802) und der zweite Kandidatenspitzenwert (801) unterschiedlich sind, nimmt der Prozessor (110) den kleineren Wert von, dem ersten Kandidatenspitzenwert (802) und dem zweiten Kandidatenspitzenwert (801) als repräsentativen Spitzenwert (802) des i-^{ten} Segmentsignals(M2).

6. Das Signalverarbeitungsverfahren gemäß Anspruch 3, wobei der Schritt des Erhaltens des repräsentativen Spitzenwerts (702, 802, 902) des i^{-ten} Segmentsignals (M1-M3) durch den Prozessor (110) unter Verwendung der dem Änderungstrend entsprechenden Berechnungsweise umfasst:
unter der Bedingung, dass der Änderungstrend der Zustand einer kontinuierlichen Abnahme ist,
Erhalten eines ersten Liniensegments (L310) durch den Prozessor (110) auf der Grundlage des ersten Spitzenwerts (601) und eines dritten Spitzenwerts (602) eines (i-1)^{-ten} Segmentsignals;
Erhalten eines zweiten Liniensegments (L320) durch den Prozessor (110) auf der Grundlage des zweiten Spitzenwerts (604) und eines vierten Spitzenwerts (603) eines (i+1)^{-ten} Segmentsignals;
Ermitteln einer Vielzahl von Kandidatenspitzenwerten (901-903) aus dem i^{ten} Segmentsignal (M3) durch den Prozessor (110);
Herausnehmen eines ersten Kandidatenspitzenwerts (902), der dem ersten Liniensegment (L310) am nächsten liegt, aus der Vielzahl von Kandidatenspitzenwerten (901-903) durch den Prozessor (110);
Durch den Prozessor (110) wird aus der Vielzahl von Kandidaten-Spitzenwerten (901-903) ein zweiter Kandidatenspitzenwert (902) ausgewählt, der dem zweiten Liniensegment (L320) am nächsten liegt.
als Reaktion darauf, dass der erste Kandidatenspitzenwert (902) und der zweite Kandidatenspitzenwert (902) identisch sind, den ersten Kandidatenspitzenwert (902) durch den Prozessor (110) als repräsentativen Spitzenwert (902) des i-^{ten} Segmentsignals(M3) zu nehmen; und
als Reaktion darauf, dass der erste Kandidatenspitzenwert und der zweite Kandidatenspitzenwert unterschiedlich sind, den größten der ersten Kandidatenspitzenwerte und des zweiten Kandidatenspitzenwertes als repräsentativen Spitzenwert des i-^{ten} Segmentsignals(M3) durch den Prozessor (110) zu nehmen.

7. Signalverarbeitungsverfahren nach einem der Ansprüche 1 bis 6, wobei der Schritt des Bestimmens durch den Prozessor (110), ob das i^{-te} Segmentsignal das gewünschte Signal oder das unerwünschte Signal ist, umfasst:
Bestimmen durch den Prozessor (110), ob das i^{-te} Segmentsignal das gewünschte Signal oder das unerwünschte Signal ist, unter Verwendung eines dynamischen Zeitverzerrungsalgorithmus.

8. Das Signalverarbeitungsverfahren gemäß einem der Ansprüche 1 bis 7, wobei nach dem Reservieren des i^{-ten} Segmentsignals (A3, A6, M1-M3), das als unerwünschtes Signal bestimmt wurde, und dem Berechnen des repräsentativen Spitzenwerts (702, 802, 902) auf der Grundlage des (i-2)^{-ten} Segmentsignals und des (i+2)^{-ten} Segmentsignals berechnet wurde, umfasst das Signalverarbeitungsverfahren ferner die folgenden Schritte:
Erhalten eines Vergleichswerts durch den Prozessor (110) auf der Grundlage eines Durchschnittswerts eines ersten Spitzenwerts des (i-2)-^{ten} Segmentsignals und eines zweiten Spitzenwerts des (i+2)- ^{ten} Segmentsignals;
Bestimmen durch den Prozessor (110), ob der repräsentative Spitzenwert (702, 802, 902) größer als der Vergleichswert ist;
als Reaktion darauf, dass der repräsentative Spitzenwert (702, 802, 902) größer als der Vergleichswert ist, Bestimmen durch den Prozessor (110), dass das i^{te} Segmentsignal (M1-M3) unzuverlässig ist, und Verwerfen des i^{ten} Segmentsignals (M1-M3); und
als Reaktion darauf, dass der repräsentative Spitzenwert (702, 802, 902) kleiner oder gleich dem Vergleichswert ist, Feststellen, dass das i-^{te} Segmentsignal (M1-M3) zuverlässig ist, und Zurückbehalten des i^{-ten} Segmentsignals (M1-M3).

9. Signalverarbeitungsvorrichtung (100), umfassend:
einen Herzschlagsensor (130), der so konfiguriert ist, dass er ein physiologisches Signal (300) eines Trägers erfasst;
eine Speichereinheit (120), die so konfiguriert ist, dass sie eine Vielzahl von Codesegmenten umfasst; und
einen Prozessor (110), der so konfiguriert ist, dass er mit dem Herzschlagsensor (130) und der Speichereinheit (120) gekoppelt werden kann, wobei der Prozessor (110) so konfiguriert ist, dass er die Vielzahl von Codesegmenten ausführt, um:
Empfangen des physiologischen Signals (300) vom Herzschlagsensor (130);
das physiologische Signal (300) auf der Grundlage einer Zeitreihe in mehrere Segmentsignale (A1-A13) zu unterteilen;
ein i^{-tes} Segmentsignal aus der Vielzahl von Segmentsignalen (A1-A13) entsprechend der Zeitreihe nacheinander herauszunehmen und zu bestimmen, ob das i-^{te} Segmentsignal ein wünschenswertes Signal oder ein unerwünschtes Signal ist;
als Reaktion darauf, dass das i^{-te} Segmentsignal (A1, A2, A4, A5, A8, A10-A13) als das wünschenswerte Signal bestimmt wurde, das i^{-te} Segmentsignal reservieren und einen Spitzenwert des reservierten i^{-ten} Segmentsignals berechnen, das als das wünschenswerte Signal bestimmt wurde;
als Reaktion darauf, dass das i^{-te} Segmentsignal (A3, A6, A7, A9) als unerwünschtes Signal bestimmt wurde, Durchführung eines Bestätigungsschritts durch den Prozessor (110), wobei der Bestätigungsschritt umfasst:
Bestimmen, ob ein (i-2)^{-tes} Segmentsignal und ein (i+2)^{-tes} Segmentsignal die gewünschten Signale sind;
Unter der Bedingung, dass sowohl das (i-2)^{-te} Segmentsignal als auch das (i+2^{)-te} Segmentsignal als wünschenswerte Signale bestimmt werden, wird das als unerwünschtes Signal bestimmte i-^{te} Segmentsignal (A3, A6, M1-M3) reserviert und ein repräsentativer Spitzenwert (702, 802, 902) auf der Grundlage des (i-2)^{-ten}-Segmentsignals und des (i+2)-^{ten}-Segmentsignals berechnet, wobei der repräsentative Spitzenwert (702, 802, 902) den Spitzenwert darstellt, der dem reservierten i-Segmentsignal (A3, A6, M1-M3) entspricht, das als unerwünschtes Signal bestimmt wurde; und
unter der Bedingung, dass mindestens eines der (i-2)-^{ten}-Segmentsignale und der (i+2)-^{ten}-Segmentsignale als unerwünschtes Signal bestimmt wird, Verwerfen des i^{-ten}-Segmentsignals(A7, A8), das als unerwünschtes Signal bestimmt wurde; und
Berechnen einer Vielzahl von instanten Herzraten (iHR1-iHR12) auf der Grundlage einer Vielzahl von Spitzenwerten (401-404, 501-504, 601-604, 702, 802, 902) der reservierten Segmentsignale.

10. Signalverarbeitungsvorrichtung (100) nach Anspruch 9, wobei der Prozessor (110) so konfiguriert ist, dass er die mehreren Codesegmente ausführt, um:
Bestimmen eines Änderungstrends des i^{-ten} Segmentsignals(M1-M3) basierend auf einem ersten Spitzenwert (401, 501, 601) des (i-2^{)-ten} Segmentsignals und einem zweiten Spitzenwert (404, 504, 604) des (i+2)^{-ten} Segmentsignals; und
Ermitteln des repräsentativen Spitzenwerts (702, 802, 902) des i-^{ten} Segmentsignals (M1-M3) unter Verwendung einer dem Änderungstrend entsprechenden Berechnungsmethode.

11. Signalverarbeitungsvorrichtung (100) nach Anspruch 10, wobei der Prozessor (110) so konfiguriert ist, dass er die mehreren Codesegmente ausführt, um:
vor der Bestimmung des Veränderungstrends des i-^{ten} Segmentsignals (M1-M3) einen Absolutwert einer Differenz zwischen einem ersten Spitzenwert (401, 501, 601) und einem zweiten Spitzenwert (404, 504, 604) zu berechnen;
den Absolutwert der Differenz mit einem Schwellenwert zu vergleichen und dadurch den Veränderungstrend des i^{-ten} Segmentsignals (M1-M3) zu bestimmen;
als Reaktion darauf, dass der Absolutwert der Differenz kleiner oder gleich dem Schwellenwert ist, Bestimmen, dass der Veränderungstrend des i-^{ten} Segmentsignals(M1) ein stabiler Zustand ist;
als Reaktion darauf, dass der Absolutwert der Differenz größer als der Schwellenwert ist und der erste Spitzenwert (501) kleiner als der zweite Spitzenwert (504) ist, wird festgestellt, dass der Veränderungstrend des i^{-ten}Segmentsignals (M2) ein kontinuierlicher Anstieg ist; und
als Reaktion darauf, dass der Absolutwert der Differenz größer als der Schwellenwert ist und der erste Spitzenwert (601) größer als der zweite Spitzenwert (604) ist, wird festgestellt, dass der Veränderungstrend des i^{-ten} Segmentsignals (M3) ein kontinuierlicher Abnahmezustand ist,
unter der Bedingung, dass der Veränderungstrend der stabile Zustand ist,
ein Liniensegment (L100) basierend auf dem ersten Spitzenwert (401) und dem zweiten Spitzenwert (404) erhalten;
Ermitteln einer Vielzahl von Kandidatenspitzenwerten (701-704) aus dem i-^{ten} Segmentsignal (M1); und
Auswählen eines der Kandidaten-Spitzenwerte (701-704), der dem Liniensegment (L100) am nächsten liegt, aus der Vielzahl von Kandidaten-Spitzenwerten (701-704) als repräsentativen Spitzenwert (702) des i-^{ten} Segmentsignals (M1),
unter der Bedingung, dass der Veränderungstrend ein kontinuierlicher Anstieg ist,
ein erstes Liniensegment (L210) auf der Grundlage des ersten Spitzenwerts (501) und eines dritten Spitzenwerts (502) eines (i-1)^{-ten} Segmentsignals erhalten;
ein zweites Liniensegment (L220) auf der Grundlage des zweiten Spitzenwerts (504) und eines vierten Spitzenwerts (503) eines (i+1)^{-ten} Segmentsignals erhalten;
Ermitteln einer Vielzahl von Kandidaten-Spitzenwerten (801, 802) aus dem i^{ten} Segmentsignal (M2);
Auswählen eines ersten Kandidatenspitzenwerts (802), der dem ersten Liniensegment (L210) am nächsten liegt, aus der Vielzahl von Kandidatenspitzenwerten (801, 802);
Auswählen eines zweiten Kandidatenspitzenwerts (802), der dem zweiten Liniensegment (L220) am nächsten liegt, aus der Vielzahl von Kandidatenspitzenwerten (801, 802);
als Reaktion darauf, dass der erste Kandidatenspitzenwert (802) und der zweite Kandidatenspitzenwert (801) gleich sind, den ersten Kandidatenspitzenwert (802) als den repräsentativen Spitzenwert (802) des i^{-ten} Segmentsignals (M2) nehmen; und
als Reaktion darauf, dass der erste Kandidatenspitzenwert (802) und der zweite Kandidatenspitzenwert (801) unterschiedlich sind, den kleineren der beiden Werte, den ersten Kandidatenspitzenwert (802) und den zweiten Kandidatenspitzenwert (801), als repräsentativen Spitzenwert (802) des i^{-ten} Segmentsignals(M2) nehmen,
unter der Bedingung, dass der Veränderungstrend ein kontinuierlicher Abfall ist,
ein erstes Liniensegment (L310) auf der Grundlage des ersten Spitzenwerts (601) und eines dritten Spitzenwerts (602) eines (i-1)^{-ten} Segmentsignals erhalten;
ein zweites Liniensegment (L320) auf der Grundlage des zweiten Spitzenwerts (604) und eines vierten Spitzenwerts (603) eines (i+1)^{-ten} Segmentsignals erhalten;
Ermitteln einer Vielzahl von Kandidatenspitzenwerten (901-903) aus dem i^{-ten} Segmentsignal (M3);
Auswählen eines ersten Kandidatenspitzenwerts (902), der dem ersten Liniensegment (L310) am nächsten liegt, aus der Vielzahl von Kandidatenspitzenwerten (901-903);
Aus der Vielzahl von Kandidatenspitzenwerten (901-903) einen zweiten Kandidatenspitzenwert (902) herausnehmen, der dem zweiten Liniensegment (L320) am nächsten liegt;
als Reaktion darauf, dass der erste Kandidatenspitzenwert (902) und der zweite Kandidatenspitzenwert (902) gleich sind, den ersten Kandidatenspitzenwert (902) als den repräsentativen Spitzenwert (902) des i^{-ten} Segmentsignals (M3) nehmen; und
als Reaktion darauf, dass der erste Kandidatenspitzenwert und der zweite Kandidatenspitzenwert unterschiedlich sind, den größten der ersten Kandidatenspitzenwerte und des zweiten Kandidatenspitzenwerts als repräsentativen Spitzenwert des i^{-ten} Segmentsignals(M3) nehmen.

12. Signalverarbeitungsvorrichtung (100) gemäß einem der Ansprüche 9 bis 11, wobei der Prozessor (110) die mehreren Codesegmente ausführt, um:
nach dem Reservieren des i^{-ten} Segmentsignals (A3, A6, M1-M3), das als unerwünschtes Signal bestimmt wurde, und dem Berechnen des repräsentativen Spitzenwerts (702, 802, 902) auf der Grundlage des (i-2)^{-ten}-Segmentsignals und des (i+2)-Segmentsignals berechnet hat, einen Vergleichswert auf der Grundlage eines Durchschnittswerts eines ersten Spitzenwerts des (i-2)^{-ten}-Segmentsignals und eines zweiten Spitzenwerts des (i+2)^{-ten}-Segmentsignals zu erhalten;
Bestimmen, ob der repräsentative Spitzenwert (702, 802, 902) größer als der Vergleichswert ist;
als Reaktion darauf, dass der repräsentative Spitzenwert (702, 802, 902) größer als der Vergleichswert ist, feststellen, dass das i-^{te} Segmentsignal (M1-M3) unzuverlässig ist, und das i-^{te} Segmentsignal (M1-M3) verwerfen; und
als Reaktion darauf, dass der repräsentative Spitzenwert (702, 802, 902) kleiner oder gleich dem Vergleichswert ist, bestimmen, dass das i^{-te} Segmentsignal (M1-M3) zuverlässig ist, und das i^{-te} Segmentsignal (M1-M3) reservieren.

13. Ein nicht-transitorisches, computerlesbares Speichermedium, wobei das nicht-transitorische, computerlesbare Speichermedium mindestens einen programmierten Befehl aufzeichnet und nachdem der mindestens eine programmierte Befehl in ein elektronisches Gerät geladen wurde, ein Prozessor (110) des elektronischen Geräts die folgenden Schritte ausführt:
Aufteilen eines physiologischen Signals (300) in eine Vielzahl von Segmentsignalen (A1-A13) auf der Grundlage einer Zeitreihe;
ein i^{-tes} Segmentsignal aus der Vielzahl von Segmentsignalen (A1-A13) entsprechend der Zeitreihe nacheinander herausnehmen und bestimmen, ob das i-^{te} Segmentsignal ein gewünschtes Signal oder ein unerwünschtes Signal ist;
als Reaktion darauf, dass das i-^{te} Segmentsignal (A1, A2, A4, A8, A10-A13) als das wünschenswerte Signal bestimmt wurde, Reservieren des i-^{ten}Segmentsignals und Berechnen eines Spitzenwerts des reservierten i-^{ten}Segmentsignals, das als das wünschenswerte Signal bestimmt wurde;
als Reaktion darauf, dass das i-^{te} Segmentsignal (A3, A6, A7, A8) als unerwünschtes Signal bestimmt wurde, Durchführen eines Bestätigungsschritts, wobei der Bestätigungsschritt umfasst:
Bestimmen, ob ein (i-2^{)-tes} Segmentsignal und ein (i+2)^{-tes} Segmentsignal die gewünschten Signale sind;
unter der Bedingung, dass sowohl das (i-2)-^{te} Segmentsignal als auch das (i+2)-^{te} Segmentsignal als die gewünschten Signale bestimmt werden, das als unerwünschtes Signal bestimmte i-^{te} Segmentsignal (A3, A6, M1-M3) reservieren und einen repräsentativen Spitzenwert (702, 802, 902) auf der Grundlage des (i-2)-^{ten} Segmentsignals und des (i+2)-^{ten}-Segmentsignals berechnet wird, wobei der repräsentative Spitzenwert (702, 802, 902) den Spitzenwert darstellt, der dem reservierten i ^{ten}-Segmentsignal (A3, A6, M1-M3) entspricht, das als unerwünschtes Signal bestimmt wurde; und
unter der Bedingung, dass bestimmt wird, dass mindestens eines der (i-2)^{-ten}-Segmentsignale und der (i+2)^{-ten}-Segmentsignale das unerwünschte Signal ist, Verwerfen des i-^{ten}-Segmentsignals(A7, A8), das als unerwünschtes Signal bestimmt wurde; und
Berechnen einer Vielzahl von instanten Herzraten (iHR1-iHR12) auf der Grundlage einer Vielzahl von Spitzenwerten (401-404, 501-504, 601-604, 702, 802, 902) der reservierten Segmentsignale.

## Revendications

1. Un Procédé de traitement de signal adapté à un dispositif électronique (100) comprenant un processeur (110), le procédé de traitement de signal comprenant les étapes suivantes :
la division d'un signal physiologique (300) par le processeur (110) en une pluralité de signaux de segment (A1-A13) sur la base d'une série chronologique ;
extraire un i-^{ème} signal de segment de la pluralité de signaux de segment (A1-A13) successivement selon la série temporelle par le processeur (110) et déterminer si le i ^{ème} signal de segment est un signal souhaité ou un signal indésirable par le processeur (110) ;
en réponse au fait que le i^{-ème} signal de segment (A1, A2, A4, A5, A8, A10-A13) a été déterminé comme étant le signal souhaité, réserver le i^{-ème} signal de segment par le processeur (110) et calculer une valeur de pic du i-^{ème} signal de segment réservé qui a été déterminé comme étant le signal souhaité ;
en réponse au fait que le i^{-ème} signal de segment (A3, A6, A7, A9) a été déterminé comme étant un signal indésirable, exécution d'une étape de confirmation par le processeur (110), l'étape de confirmation comprenant :
déterminer si un (i-2) ^{ème} signal de segment et un (i+2) ^{ème} signal de segment sont les signaux souhaités ;
à condition que le (i-2)- ^{ème} signal de segment et le (i+2^{)- ème} signal de segment soient tous deux déterminés comme étant des signaux souhaitables, le i^{-ème} (A3, A6, M1-M3) est réservé et une valeur de pic représentative (702, 802, 902) est calculée sur la base du (i-2) ^{-ème} signal de segment et du (i+2) ^{-ème}, la valeur de pic représentative (702, 802, 902) représentant la valeur de pic correspondant au i-^{ème} signal de segment (A3, A6, M1-M3) retenu qui a été déterminé comme étant un signal indésirable ; et
à condition qu'au moins l'un des (i-2)^{-ème} signaux de segment et des (i+2)^{-ème} signaux de segment soit déterminé comme signal indésirable, rejeter le i^{-ème} signal de segment (A7, A9) qui a été déterminé comme signal indésirable ; et
calculer une pluralité de fréquences cardiaques instantanées (iHR1-iHR12) par le processeur (110) sur la base d'une pluralité de valeurs de pic (401-404, 501-504, 601-604, 702, 802, 902) des signaux de segment réservés.

2. Le procédé de traitement de signal selon la revendication 1, dans lequel l'étape consistant à calculer la valeur de pic représentative (702, 802, 902) sur la base du (i-2)-^{ème} signal de segment et du (i+2) ^{-ème} signal de segment comprend les étapes suivantes :
détermination d'un taux de variation du i-^{ème} signal de segment (M1-M3) par le processeur (110) sur la base d'une première valeur de pic (401, 501, 601) du (i-2)^{-ème} et d'une deuxième valeur de pic (404, 504, 604) du signal de segment (i+2) ^{-ème} ; et
détermination de la valeur de pic représentative (702, 802, 902) du i-^{ème} signal de segment (M1-M3) par le processeur (110) à l'aide d'une méthode de calcul correspondant à la tendance de variation.

3. Le procédé de traitement de signal selon la revendication 2, dans lequel, avant l'étape de détermination de la tendance de changement du i^{-ème} signal de segment (M1-M3) par le processeur (110), le procédé de traitement de signal comprend en outre les étapes suivantes :
calculer une valeur absolue d'une différence entre la première valeur de pic d' (401, 501, 601) et la deuxième valeur de pic (404, 504, 604) par le processeur (110) ;
l'étape de détermination de la variation du i^{-ème} signal de segment (M1-M3) par le processeur (110) comprenant :
la comparaison de la valeur absolue de la différence avec un seuil ;
en réponse au fait que la valeur absolue de la différence est inférieure ou égale au seuil, la détermination par le processeur (110) que la tendance de variation du i^{-ème} signal de segment (M1) est un état stable ;
en réponse au fait que la valeur absolue de la différence est supérieure au seuil et que la première valeur de pic (501) est inférieure à la deuxième valeur de pic (504), le processeur (110) détermine que la tendance de variation du i^{-ème} signal de segment (M2) est un état d'augmentation continue ; et
en réponse au fait que la valeur absolue de la différence est supérieure au seuil et que la première valeur de pic (601) est supérieure à la deuxième valeur de pic (604), le processeur (110) détermine que la tendance de variation du i^{-ème} signal de segment (M3) est un état de diminution continue.

4. Le procédé de traitement de signal selon la revendication 3, dans lequel l'étape consistant à obtenir la valeur de pic représentative (702, 802, 902) du i^{-ème} signal de segment (M1-M3) par le processeur (110) en utilisant le mode de calcul correspondant à la tendance de changement comprend :
à condition que la tendance de variation soit l'état stable,
obtention d'un segment de ligne (L100) par le processeur (110) sur la base de la première valeur de pic (401) et de la deuxième valeur de pic (404) ;
détermination d'une pluralité de valeurs de pic candidates (701-704) à partir d' du i-^{ème} signal de segment (M1) par le processeur (110) ; et
sélectionner l'une des valeurs de pic candidates (701-704) la plus proche du segment de ligne (L100) parmi la pluralité de valeurs de pic candidates (701-704) en tant que valeur de pic représentative (702) du i-^{ème} signal de segment (M1) par le processeur (110).

5. Le procédé de traitement de signal selon la revendication 3, dans lequel l'étape consistant à obtenir la valeur de pic représentative (702, 802, 902) du i-^{ème} signal de segment(M1-M3) par le processeur (110) en utilisant le mode de calcul correspondant à la tendance de changement comprend :
à condition que la tendance de changement soit un état d'augmentation continue,
obtention d'un premier segment de ligne (L210) par le processeur (110) sur la base de la première valeur de pic (501) et d'une troisième valeur de pic (502) d'un (i-1) ^{ème} signal de segment ;
obtention d'un deuxième segment de ligne (L220) par le processeur (110) sur la base de la deuxième valeur de pic (504) et d'une quatrième valeur de pic (503) d'un (i+1)^{-ème} signal de segment ;
détermination d'une pluralité de valeurs de pic candidates (801, 802) à partir du i^{-ème} signal de segment (M2) par le processeur (110) ;
retrait d'une première valeur de pic candidate (802) la plus proche du premier segment de ligne (L210) parmi la pluralité de valeurs de pic candidates (801, 802) par le processeur (110) ;
sélectionner une deuxième valeur de pic candidate (801) la plus proche du deuxième segment de ligne (L220) parmi la pluralité d' s de valeurs de pic candidates (801, 802) par le processeur (110) ;
en réponse au fait que la première valeur de pic candidate (802) et la deuxième valeur de pic candidate (801) sont identiques, prendre la première valeur de pic candidate (802) comme valeur de pic représentative (802) du i-^{ème} signal de segment (M2) par le processeur (110) ; et
en réponse au fait que la première valeur de pic candidate (802) et la deuxième valeur de pic candidate (801) sont différentes, le processeur (110) prend la plus petite valeur parmi la première valeur de pic candidate (802) et la deuxième valeur de pic candidate (801) comme valeur de pic représentative (802) du i^{-ème} signal de segment (M2).

6. Le procédé de traitement de signal selon la revendication 3, dans lequel l'étape consistant à obtenir la valeur de pic représentative (702, 802, 902) du i-^{ème} signal de segment(M1-M3) par le processeur (110) en utilisant le mode de calcul correspondant à la tendance de changement comprend :
à condition que la tendance de changement soit un état de diminution continue,
obtention d'un premier segment de ligne (L310) par le processeur (110) sur la base de la première valeur de pic (601) et d'une troisième valeur de pic (602) d'un (i-1) ^{-ème} signal de segment ;
obtention d'un deuxième segment de ligne (L320) par le processeur (110) sur la base de la deuxième valeur de pic (604) et d'une quatrième valeur de pic (603) d'un (i+1)^{-ème} signal de segment ;
détermination d'une pluralité de valeurs de pic candidates (901-903) à partir du i-^{ème} signal de segment (M3) par le processeur (110) ;
extraction par le processeur (110) d'une première valeur de pic candidate (902) la plus proche du premier segment de ligne (L310) à partir de la pluralité de valeurs de pic candidates (901-903) ;
Le processeur (110) sélectionne, parmi la pluralité de valeurs de pic candidates (901-903), une deuxième valeur de pic candidate (902) qui est la plus proche du deuxième segment de ligne (L320).
en réponse au fait que la première valeur de pic candidate (902) et la deuxième valeur de pic candidate (902) sont identiques, le processeur (110) prend la première valeur de pic candidate (902) comme valeur de pic représentative (902) du i-^{ème} signal de segment (M3) ; et
en réponse au fait que la première valeur de pic candidate et la deuxième valeur de pic candidate sont différentes, prendre la plus grande des première et deuxième valeurs de pic candidates comme valeur de pic représentative du i-^{ème} signal de segment (M3) par le processeur (110).

7. Procédé de traitement de signal selon l'une des revendications 1 à 6, dans lequel l'étape consistant pour le processeur (110) à déterminer si le i^{-ème} signal de segment est le signal souhaité ou le signal indésirable comprend :
déterminer par le processeur (110) si le i^{-ème} signal de segment est le signal souhaité ou le signal indésirable en utilisant un algorithme de distorsion temporelle dynamique.

8. Procédé de traitement de signal selon l'une des revendications 1 à 7, dans lequel, après avoir réservé le i-^{ème} signal de segment (A3, A6, M1-M3) qui a été déterminé comme étant un signal indésirable et avoir calculé la valeur de pic représentative (702, 802, 902) sur la base du (i-2)^{-ème} signal de segment et du (i+2)^{-ème} signal de segment, le procédé de traitement de signal comprend en outre les étapes suivantes :
obtention d'une valeur de comparaison par le processeur (110) sur la base d'une valeur moyenne d'une première valeur de pic du (i-2)^{-ème} signal de segment et d'une deuxième valeur de pic du (i+2)^{-ème} signal de segment ;
détermination par le processeur (110) si la valeur de pic représentative (702, 802, 902) est supérieure à la valeur de comparaison ;
en réponse au fait que la valeur de pic représentative (702, 802, 902) est supérieure à la valeur de comparaison, détermination par le processeur (110) que le i^{- ème} signal de segment (M1-M3) n'est pas fiable et rejet du i^{-ème} signal de segment (M1-M3) ; et
en réponse au fait que la valeur de pic représentative (702, 802, 902) est inférieure ou égale à la valeur de comparaison, déterminer que le i^{-ème} signal de segment (M1-M3) est fiable et conserver le i^{-ème} signal de segment (M1-M3).

9. Dispositif de traitement de signal (100) comprenant :
un capteur de battements cardiaques (130) configuré pour détecter un signal physiologique (300) d'un porteur ;
une unité de mémoire (120) configurée pour comprendre une pluralité de segments de code ; et
un processeur (110) configuré pour être couplé au capteur de battements cardiaques (130) et à l'unité de mémoire (120), le processeur (110) étant configuré pour exécuter la pluralité de segments de code afin de :
recevoir le signal physiologique (300) provenant du capteur de battements cardiaques (130) ;
diviser le signal physiologique (300) en plusieurs signaux de segment d' s (A1-A13) sur la base d'une série chronologique ;
extraire successivement un i^{-ème} signal de segment de la pluralité de signaux de segment (A1-A13) correspondant à la série temporelle et déterminer si le i^{-ème} signal de segment est un signal souhaitable ou un signal indésirable ;
en réponse au fait que le i^{-ème} signal de segment (A1, A2, A4, A5, A8, A10-A13) a été déterminé comme étant le signal souhaitable, réserver le i^{-ème} signal de segment et calculer une valeur de pic du i^{-ème} signal de segment réservé qui a été déterminé comme étant le signal souhaitable ;
en réponse au fait que le i^{-ème} signal de segment (A3, A6, A7, A9) a été déterminé comme étant un signal indésirable, exécution d'une étape de confirmation par le processeur (110), l'étape de confirmation comprenant :
déterminer si un (i-2)^{-ème} signal de segment et un (i+2)^{-ème} signal de segment sont les signaux souhaités ;
à condition que le (i-2)^{-ème} signal de segment et le (i+2)^{-ème} signal de segment sont déterminés comme étant des signaux souhaitables, le signal de segment i^{-(te)} (A3, A6, M1-M3) déterminé comme étant un signal indésirable est réservé et une valeur de pic représentative (702, 802, 902) est calculée sur la base du (i-2)^{-ème} signal de segment et le (i+2)^{-(te)n} signal de segment, la valeur de pic représentative (702, 802, 902) représentant la valeur de pic correspondant au signal de segment i réservé (A3, A6, M1-M3) qui a été déterminé comme signal indésirable ; et
à condition qu'au moins l'un des (i-2)^{-ème} signaux de segment et des (i+2)^{-ème} signaux de segment soit déterminé comme signal indésirable, rejeter le i^{-ème} signal de segment (A7, A8) qui a été déterminé comme signal indésirable ; et
Calculer une pluralité de fréquences cardiaques instantanées (iHR1-iHR12) sur la base d'une pluralité de valeurs de pic (401-404, 501-504, 601-604, 702, 802, 902) des signaux de segment réservés.

10. Dispositif de traitement de signal (100) selon la revendication 9, dans lequel le processeur (110) est configuré pour exécuter les multiples segments de code afin de :
déterminer une tendance de changement du i^{-ème} signal de segment (M1-M3) sur la base d'une première valeur de pic (401, 501, 601) du (i-2) ^{-ème} signal de segment et d'une deuxième valeur de pic (404, 504, 604) du (i+2) ^{-ème} signal de segment ; et
déterminer la valeur de pic représentative (702, 802, 902) du i^{-ème} signal de segment (M1-M3) en utilisant une méthode de calcul correspondant à la tendance de variation.

11. Dispositif de traitement de signal (100) selon la revendication 10, dans lequel le processeur (110) est configuré pour exécuter les multiples segments de code afin de :
avant de déterminer la tendance de variation du i-^{ème} signal de segment (M1-M3), calculer une valeur absolue d'une différence entre une première valeur de pic (401, 501, 601) et une deuxième valeur de pic (404, 504, 604) ;
comparer la valeur absolue de la différence à un seuil et déterminer ainsi la tendance de variation du i-^{ème} signal de segment (M1-M3) ;
en réponse au fait que la valeur absolue de la différence est inférieure ou égale au seuil, déterminer que la tendance de variation du i-^{ème} signal de segment (M1) est un état stable ;
en réponse au fait que la valeur absolue de la différence est supérieure au seuil et que la première valeur de pic (501) est inférieure à la deuxième valeur de pic (504), déterminer que la tendance de variation du i^{-ème} signal de segment (M2) est une augmentation continue e ; et
en réponse au fait que la valeur absolue de la différence est supérieure au seuil et que la première valeur de pic (601) est supérieure à la deuxième valeur de pic (604), il est déterminé que la tendance de variation du i ^{ème -} signal de segment (M3) est un état de diminution continue,
à condition que la tendance de variation soit l'état stable,
un segment de ligne (L100) basé sur la première valeur de pic (401) et la deuxième valeur de pic (404) ;
déterminer une pluralité de valeurs de pic candidates (701-704) à partir du i-^{ème} signal de segment (M1) ; et
sélectionner l'une des valeurs de pic candidates (701-704) la plus proche du segment de ligne (L100) parmi la pluralité de valeurs de pic candidates (701-704) en tant que valeur de pic représentative (702) du i-^{ème} signal de segment (M1),
à condition que la tendance de variation soit une augmentation continue,
obtenir un premier segment de ligne (L210) sur la base de la première valeur de pic (501) et d'une troisième valeur de pic (502) d'un (i-1) ^{-ème} signal de segment ;
obtenir un deuxième segment de ligne (L220) sur la base de la deuxième valeur de pic (504) et d'une quatrième valeur de pic (503) d'un (i+1) ^{-ème} signal de segment ;
détermination d'une pluralité de valeurs de pic candidates (801, 802) à partir du i^{-ème} signal de segment (M2) ;
sélectionner une première valeur de pic candidate (802) la plus proche du premier segment de ligne (L210) parmi la pluralité de valeurs de pic candidates (801, 802) ;
sélectionner une deuxième valeur de pic candidate (802) la plus proche du deuxième segment de ligne (L220) parmi la pluralité de valeurs de pic candidates (801, 802) ;
en réponse au fait que la première valeur de pic candidate (802) et la deuxième valeur de pic candidate (801) sont identiques, prendre la première valeur de pic candidate (802) comme valeur de pic représentative (802) du i-^{ème} signal de segment (M2) ; et
en réponse au fait que la première valeur de pic candidate (802) et la deuxième valeur de pic candidate (801) sont différentes, prendre la plus petite des deux valeurs, la première valeur de pic candidate (802) et la deuxième valeur de pic candidate (801), comme valeur de pic représentative (802) du i-^{ème} signal de segment (M2),
à condition que la tendance de variation soit une baisse continue,
obtenir un premier segment de ligne (L310) sur la base de la première valeur de pic (601) et d'une troisième valeur de pic (602) d'un (i-1) ^{ème} signal de segment ;
obtenir un deuxième segment de ligne (L320) sur la base de la deuxième valeur de pic (604) et d'une quatrième valeur de pic (603) d'un (i+1) ^{-ème} signal de segment ;
détermination d'une pluralité de valeurs de pic candidates (901-903) à partir du i^{-ème} signal de segment (M3) ;
sélectionner une première valeur de pic candidate (902) la plus proche du premier segment de ligne (L310) parmi la pluralité de valeurs de pic candidates (901-903) ;
Retirer de la pluralité de valeurs de pic candidates (901-903) une deuxième valeur de pic candidate (902) qui est la plus proche du deuxième segment de ligne (L320) ;
en réponse au fait que la première valeur de pic candidate (902) et la deuxième valeur de pic candidate (902) sont identiques, prendre la première valeur de pic candidate (902) comme valeur de pic représentative (902) du i-^{ème} signal de segment (M3) ; et
en réponse au fait que la première valeur de pic candidate et la deuxième valeur de pic candidate sont différentes, prendre la plus grande des première et deuxième valeurs de pic candidates comme valeur de pic représentative du i-^{ème} signal de segment (M3).

12. Dispositif de traitement de signal (100) selon l'une des revendications 9 à 11, dans lequel le processeur (110) exécute les multiples segments de code pour :
après avoir réservé le i-^{ème} signal de segment (A3, A6, M1-M3) qui a été déterminé comme étant un signal indésirable et avoir calculé la valeur de pic représentative (702, 802, 902) sur la base du (i-2) ^{-ème} signal de segment et du (i+2)^{-ème} signal de segment, obtenir une valeur de comparaison sur la base d'une valeur moyenne d'une première valeur de pic du (i-2)^{-ème} signal de segment et d'une deuxième valeur de pic du (i+2) ^{ème} signal de segment ;
déterminer si la valeur de pic représentative (702, 802, 902) est supérieure à la valeur de comparaison ;
en réponse au fait que la valeur de pic représentative (702, 802, 902) est supérieure à la valeur de comparaison, déterminer que le i ^{-ème} signal de segment (M1-M3) n'est pas fiable et rejeter le i^{-ème} signal de segment (M1-M3) ; et
en réponse au fait que la valeur de pic représentative (702, 802, 902) est inférieure ou égale à la valeur de comparaison, déterminer que le signal de segment i^{-ème} (M1-M3) est fiable et réserver le signal de segment i^{-ème}(M1-M3).

13. Support de stockage non transitoire lisible par ordinateur, le support de stockage non transitoire lisible par ordinateur enregistrant au moins une instruction programmée et, après que la au moins une instruction programmée a été chargée dans un dispositif électronique, un processeur (110) du dispositif électronique exécutant les étapes suivantes :
diviser un signal physiologique (300) en une pluralité de signaux de segment (A1-A13) sur la base d'une série chronologique ;
extraire successivement un i^{-ème} signal de segment de la pluralité de signaux de segment (A1-A13) correspondant à la série chronologique et déterminer si le i-^{ème} signal de segment est un signal souhaité ou un signal indésirable ;
en réponse au fait que le i^{-ème} signal de segment (A1, A2, A4, A8, A10-A13) a été déterminé comme étant le signal souhaité, réserver le i^{-ème} signal de segment et calculer une valeur de pic du i^{-ème} signal de segment réservé qui a été déterminé comme étant le signal souhaité ;
en réponse au fait que le i^{-ème} signal de segment (A3, A6, A7, A8) a été déterminé comme étant un signal indésirable, exécution d'une étape de confirmation, l'étape de confirmation comprenant :
déterminer si un (i-2)^{-ème} signal de segment et un (i+2)^{-ème} signal de segment sont les signaux souhaités ;
à condition que le (i-2)-^{ème} signal de segment et le (i+2)-^{ème} signal de segment soient tous deux déterminés comme étant les signaux souhaités, le (i-2)^{ème} signal de segment déterminé comme étant un signal indésirable sont déterminés comme étant les signaux souhaités, réserver le signal de segment i^{-ème} (A3, A6, M1-M3) déterminé comme étant un signal indésirable et calculer une valeur de pic représentative (702, 802, 902) sur la base du signal de segment (i-2)^{-ème} signal de segment et du (i+2)^{-ème} signal de segment, la valeur de pic représentative (702, 802, 902) représentant la valeur de pic correspondant au signal de segment i^{-ème} réservé (A3, A6, M1-M3) qui a été déterminé comme signal indésirable ; et
à condition qu'il soit déterminé qu'au moins l'un des (i-2) ^{-ème} signaux de segment et des (i+2) ^{-ème} signaux de segment est le signal indésirable, rejeter le signal de segment i-^{-ème} (A7, A8) qui a été déterminé comme étant le signal indésirable ; et
calculer une pluralité de fréquences cardiaques instantanées (iHR1-iHR12) sur la base d'une pluralité de valeurs de pic (401-404, 501-504, 601-604, 702, 802, 902) des signaux de segment réservés.
